(19) 

Europäisches
Patentamt
European
Patent Office
Office européen
des brevets

(11) **EP 4 410 836 A1**

(12) **EUROPEAN PATENT APPLICATION**
published in accordance with Art. 153(4) EPC

(43) Date of publication:
**07.08.2024 Bulletin 2024/32**

(21) Application number: **22875057.6**

(22) Date of filing: **29.09.2022**

(51) International Patent Classification (IPC):
*C07K 16/28* (2006.01)     *A61K 39/395* (2006.01)
*A61P 35/00* (2006.01)

(52) Cooperative Patent Classification (CPC):
**A61K 39/395; A61P 35/00; C07K 16/28**

(86) International application number:
**PCT/CN2022/122513**

(87) International publication number:
**WO 2023/051674 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.09.2021 CN 202111163030**

(71) Applicants:
• **Nanjing Shunxin Pharmaceuticals Co., Ltd.
of Chiatai Tianqing Pharmaceutical Group
Nanjing, Jiangsu 211100 (CN)**
• **CHIA TAI TIANQING PHARMACEUTICAL GROUP
CO., LTD.
Lianyungang,
Jiangsu 222062 (CN)**

(72) Inventors:
• **YANG, Anqi
Nanjing, Jiangsu 211100 (CN)**

• **ZHANG, Xiquan
Nanjing, Jiangsu 211100 (CN)**
• **YU, Ding
Nanjing, Jiangsu 211100 (CN)**
• **LV, Peng
Nanjing, Jiangsu 211100 (CN)**
• **WANG, Xunqiang
Nanjing, Jiangsu 211100 (CN)**
• **TIAN, Xin
Nanjing, Jiangsu 211100 (CN)**
• **CHEN, Qin
Nanjing, Jiangsu 211100 (CN)**

(74) Representative: **v. Bezold & Partner Patentanwälte
- PartG mbB
Ridlerstraße 57
80339 München (DE)**

Remarks:
The complete document including Reference
Table(s) and the Sequence Listing(s) can be
downloaded from the EPO website

(54) **ANTI-CD47 ANTIBODY FOR COMBINED TREATMENT OF BLOOD TUMOR**

(57)     An anti-CD47 antibody for combined treatment of a blood tumor. Provided are the use of the anti-CD47 antibody and a second therapeutic agent in the preparation of a drug for treating a blood tumor in a subject. Provided is a method for treating a blood tumor in a subject, comprising administering a therapeutically effective amount of the anti-CD47 antibody and the second therapeutic agent to a subject. Also provided are a pharmaceutical combination comprising the anti-CD47 antibody and the second therapeutic agent, and a kit.

FIG. 8

**Description**

**TECHNICAL FIELD**

The present disclosure belongs to the field of biological medicines, and relates to use of an anti-CD47 antibody and a second therapeutic agent for combating a hematologic tumor, and a pharmaceutical combination.

**BACKGROUND**

CD47, also known as integrin-associated protein, is a transmembrane glycoprotein widely expressed among a variety of species and tissues. It is a member of the immunoglobulin superfamily. Inhibitory receptor signal-regulatory protein α (SIRPα) is one of the ligands of CD47, which binds to the $NH_2$-terminal IgV-like domain of SIRPα. SIRPα is expressed primarily in myeloid-derived cells, including macrophages, granulocytes, DC cells, mast cells, and their precursors, including hematopoietic stem cells. CD47 expression and/or activity has been implicated in a number of diseases and disorders. Several different studies have shown that almost all tumor cells and tumor tissues highly express CD47. CD47 highly expressed on the tumor cell surface binds to SIRPα on the macrophage surface, sending a "don't eat me" signal, so that the macrophages in the tumor tissue infiltration area get along with the tumor cells and even facilitate the expansion and growth of the tumor cells by facilitating the proliferation of blood vessels in tumors and suppressing effector T cells. Drugs targeting the CD47-SIRPα axis, such as anti-CD47 antibodies, are currently in clinical trials.

At present, there is still an unmet clinical need for the treatment of cancer, particularly a hematologic tumor.

**SUMMARY**

The present disclosure provides use of an anti-CD47 antibody and a second therapeutic agent for preparing a medicament for treating a hematologic tumor in a subject.

The present disclosure provides a method for treating a hematologic tumor in a subject, comprising administering to the subject a therapeutically effective amount of an anti-CD47 antibody and a second therapeutic agent.

The present disclosure also provides a pharmaceutical combination comprising an anti-CD47 antibody and a second therapeutic agent.

The present disclosure also provides a pharmaceutical combination for treating a hematologic tumor, comprising an anti-CD47 antibody and a second therapeutic agent.

In some embodiments, the second therapeutic agent described above is selected from the group consisting of a hypomethylating agent, an anti-CD20 antibody, an immunomodulator, and a proteasome inhibitor.

In some embodiments, the anti-CD47 antibody is the anti-CD47 antibody provided herein.

The present disclosure also provides a kit comprising the pharmaceutical combination.

The present disclosure provides use of the anti-CD47 antibody herein for preparing a medicament for treating myeloma in a subject. Also provided is a method for treating myeloma in a subject, comprising administering to the subject a therapeutically effective amount of the anti-CD47 antibody herein.

**BRIEF DESCRIPTION OF THE DRAWINGS**

FIG. 1 shows the effects of anti-CD47 antibodies on the survival rate of mice.

FIG. 2 shows the change in body weight of mice over time after single administration of anti-CD47 antibodies.

FIG. 3 shows the rate of change in body weight of mice at different time points after single administration of anti-CD47 antibodies.

FIG. 4 shows the change in RBC of mice over time after single administration of anti-CD47 antibodies.

FIG. 5 shows the rate of change in RBC of mice at different time points after single administration of anti-CD47 antibodies.

FIG. 6 shows the change in HGB content of mice over time after single administration of anti-CD47 antibodies.

FIG. 7 shows the rate of change in HGB content of mice at different time points after single administration of anti-CD47 antibodies.

FIG. 8 shows the change in tumor volume of subcutaneous mouse xenograft tumor of human acute myeloblastic leukemia MOLM-16 after administration of an anti-CD47 antibody and azacitadine alone or in combination.

FIG. 9 shows the change in tumor volume of subcutaneous mouse xenograft tumor of human B-cell lymphoma Daudi after administration of an anti-CD47 antibody and rituximab alone or in combination.

DETAILED SUMMARY

Terminology

The term "antibody" is used in its broadest sense and encompasses a variety of antibody structures, including natural and artificial antibodies of various structures, including, but not limited to, monoclonal antibodies, monospecific antibodies, multispecific antibodies (e.g., bispecific antibodies, trispecific antibodies, etc.), single-chain antibodies, antibody fragments, and the like, so long as they exhibit the desired antigen-binding activity. The antibody may be an intact antibody. An antibody fragment refers to a molecule other than an intact antibody, which comprises a portion of the intact antibody that binds to the antigen to which the intact antibody binds. Examples of antibody fragments include, but are not limited to, Fv, Fab, Fab', Fab'-SH, F(ab')$_2$, single-chain antibody molecules (e.g., scFv), and single-domain antibodies.

The term "isotype" refers to the class of antibodies encoded by the heavy chain constant region gene.

The term "chimeric antibody" refers to an antibody having at least a portion of a heavy chain variable region and a portion of a light chain variable region derived from one species, and at least a portion of a constant region derived from another species. For example, in some embodiments, the chimeric antibody may comprise a murine variable region and a human constant region.

The term "humanized antibody" refers to an antibody comprising complementarity determining regions (CDRs) derived from a non-human antibody, and framework and constant regions derived from a human antibody. For example, the humanized antibody that binds to CD47 provided herein may comprise CDRs derived from one or more murine antibodies and human framework and constant regions.

The term "variable domain" or "variable region" refers to a domain of an antibody that is involved in the binding of the antibody to an antigen. For example, a natural four-chain antibody (e.g., derived from human, murine, and the like) has a heavy chain variable region (VH) and a light chain variable region (VL), and a heavy-chain antibody derived from an animal such as camelid or shark has a single variable domain. In most cases, each variable domain of a natural antibody consists substantially of four "framework regions" and three "complementarity determining regions". The four framework regions are referred to as framework region 1 (FR1), framework region 2 (FR2), framework region 3 (FR3), and framework region 4 (FR4), respectively. The framework regions are separated by three complementarity determining regions (CDRs) referred to in the art and hereinafter as complementarity determining region 1 (CDR1), complementarity determining region 2 (CDR2), and complementarity determining region 3 (CDR3), respectively. Thus, the general structure of a variable domain can be represented as follows: FR1-CDR1-FR2-CDR2-FR3-CDR3-FR4. In one specific example, the general structure of a heavy chain variable region can be represented as follows: FR1-HCDR1-FR2-HCDR2-FR3-HCDR3-FR4. In one specific example, the general structure of a light chain variable region can be represented as follows: FR1-LCDR1-FR2-LCDR2-FR3-LCDR3-FR4. Variable domains impart specificity for an antigen to an antibody by virtue of having an antigen-binding site.

"CDR" (complementarity determining region), also referred to "hypervariable region (HVR)", typically refers to each region of antibody variable regions which are highly variable in sequence and/or form structurally defined loops. A natural four-chain antibody typically comprises six CDRs, among which three are in the heavy chain variable region, i.e., heavy chain CDR1 (HCDR1), heavy chain CDR2 (HCDR2) and heavy chain CDR3 (HCDR3), and three are in the light chain variable region, i.e., light chain CDR1 (LCDR1), light chain CDR2 (LCDR2) and light chain CDR3 (LCDR3). A heavy-chain antibody or a single variable domain typically has three CDRs (CDR1, CDR2, and CDR3).

There are currently many ways to define CDRs. The Kabat scheme defines CDRs based on the sequence variability and is the most commonly used (Elvin A. Kabat, et al., Sequences of Proteins of Immunological Interest, 5th Edition, Public Health Service, National Institutes of Health, Bethesda, Md. (1991)), while the Chothia scheme defines CDRs based on the positions of structural loops (Cyrus Chothia, et al., Canonical Structures for the Hypervariable Regions of Immunoglobulins, J. Mol. Biol. 196:901-917(1987)). The AbM scheme, a compromise between the Kabat scheme and the Chothia scheme, is used by the Oxford Molecular's AbM antibody modeling software. "Contact" defines CDRs based on the analysis of the crystal structure of available complexes. However, it should be noted that boundaries of the CDRs of variable regions of the same antibody based on different methods and definitions may differ, that is, CDR sequences of the variable regions of the same antibody defined by different methods may differ. Thus, when reference is made to an antibody defined with a particular CDR sequence defined herein, the scope of the antibody also encompasses antibodies defined by CDR sequences that are converted to other arbitrary definitions (e.g., Chothia, AbM definitions, etc.).

The term "framework region" (FR) refers to amino acid residues of variable domains other than the CDR residues defined herein.

The term "isolated" means that a target compound (e.g., an antibody or nucleic acid) has been isolated from its natural environment.

As used herein, the term "EC$_{50}$" refers to the effective concentration, 50% of the maximal response of an antibody. As used herein, the term "IC$_{50}$" refers to the inhibitory concentration, 50% of the maximal response of an

antibody. Both $EC_{50}$ and $IC_{50}$ can be measured by ELISA or FACS assay or any other method known in the art. The term "$K_D$" as used herein refers to the equilibrium dissociation constant, expressed in molar concentration (M). The $K_D$ value of an antibody can be determined using methods well known in the art. One preferred method for determining the $K_D$ of an antibody is performed by using surface plasmon resonance, more preferably a biosensor system, such as a Biacore system.

As used herein, the term "subject" includes any human or non-human animal. The term "non-human animal" includes all vertebrates, e.g., mammals and non-mammals, such as non-human primates, sheep, dogs, cats, horses, cows, chickens, amphibians, and reptiles. Preferably, the subject according to the present disclosure is a human. The terms "patient" and "subject" are used interchangeably unless otherwise indicated.

The terms "hematologic malignancy", "hematologic tumor", and "hematologic cancer" are used interchangeably herein in the broadest sense and refer to all stages and forms of cancer caused by cells of the hematopoietic system.

The term "treating" or "treatment" refers to an attempt to alter the natural course of a disease in a treated individual, and may be a clinical intervention performed for prophylaxis or during the course of clinical pathology. Desired therapeutic effects include, but are not limited to, preventing the occurrence or recurrence of diseases, relieving symptoms, reducing any direct or indirect pathological outcomes of diseases, preventing metastasis, slowing disease progression rates, improving or alleviating disease conditions, and regressing or improving prognosis.

As used herein, the term "therapeutically effective amount" refers to the amount of a compound, a composition, or a pharmaceutical combination necessary to provide a therapeutic benefit to a subject.

The term "non-fixed combination" refers to simultaneous, parallel, or sequential and temporally unlimited administration of two or more active components as independent entities (for example, pharmaceutical compositions and formulations) to a subject, wherein the active ingredients administered to the subject reach therapeutically effective amounts. An example, which can be listed, of the non-fixed combination is a cocktail therapy, for example, 3 or more active components are administered. In a non-fixed combination, each of the active components can be packaged, sold or administered as a fully independent pharmaceutical composition.

The term "fixed dose" refers to a dose administered to a patient without considering the body weight or the body surface area (BSA) of the patient. Thus, the fixed dose is specified as an absolute amount of an agent (e.g., anti-CD47 antibody) rather than, for example, an mg/kg dose. For example, a human of 80 kg and a human of 100 kg will receive the same dose of antibody (e.g., 800 mg of the anti-CD47 antibody). In a manner different from the fixed dose, it is also possible to perform administration in a dose calculated in accordance with the body weight or body surface area of the patient, for example, in a dose of 0.5 mg/kg to 70 mg/kg in accordance with the body weight of the patient.

The term "single dose" refers to the smallest unit of packaging containing a certain quantity of pharmaceutical product; for example, in a box of seven capsules, each capsule is a single dose; or a vial of injection is a single dose. As used herein, the terms "single dose" and "unit dose" have the same meaning and are used interchangeably.

"Pharmaceutical composition" refers to a composition comprising an active ingredient and a pharmaceutically acceptable carrier.

The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without any conservative replacements as part of the sequence identity. Alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to obtain maximum alignment for the full length of sequences being compared.

The terms "Xn" and "Xaa" are equivalent and refer to an unspecified amino acid whose scope is specified by the subsequent definitions in the relevant description.

The term "include", "contain", or "comprise" and variants thereof should be understood as "including, but not limited to", which means that other elements, components and steps that are not specified are encompassed in addition to the elements, components and steps that are listed.

Unless otherwise specified clearly herein, singular terms encompass plural terms, and vice versa.

All patents, patent applications and other identified publications are explicitly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art. Various aspects of the present disclosure will be described in further detail in the following sections.

The anti-CD47 antibody provided herein is suitable for treatment of hematologic tumors, alone or in combination with other therapeutic agents. In addition, the combination described above may also have one or several of the following advantages:

(1) In some experiments, it was found that the combination of the anti-CD47 antibody and other therapeutic agents (e.g., anti-CD20 antibodies, hypomethylating agents, etc.) can enhance the anti-tumor effect of a single agent, e.g., killing tumor cells, inhibiting tumor growth, or/and eliminating tumors, etc. In particular, for example, in some hematologic tumors such as lymphomas, leukemias, myelodysplastic syndromes, multiple myelomas, etc., it has good efficacy or/and safety.
(2) The combination of the anti-CD47 antibody provided herein and other therapeutic agents can provide a treatment that is well tolerated in a subject with good safety, e.g., with fewer adverse reactions or/and complications.
(3) The combination of the anti-CD47 antibody provided herein and other therapeutic agents can allow for administration in smaller amounts (e.g., administration amounts or/and total administration amounts) and benefit from the administration as compared to a single agent or some other known agents or combination of agents.

### Combination of Anti-CD47 Antibody and Second Therapeutic Agent and Use

The present disclosure provides use of an anti-CD47 antibody and a second therapeutic agent for preparing a medicament for treating a hematologic tumor in a subject.

The present disclosure provides a method for treating a hematologic tumor in a subject, comprising administering to the subject a therapeutically effective amount of an anti-CD47 antibody and a second therapeutic agent.

In some embodiments, the anti-CD47 antibody is selected from the group consisting of those described in the section of *"Anti-CD47 Antibody"*. In some specific embodiments, the anti-CD47 antibody comprises: a heavy chain variable region comprising: HCDR1 comprising an amino acid sequence set forth in SEQ ID NO: 5, HCDR2 comprising an amino acid sequence set forth in SEQ ID NO: 10, and HCDR3 comprising an amino acid sequence set forth in SEQ ID NO: 15; and a light chain variable region comprising: LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30. In some specific embodiments, the anti-CD47 antibody comprises a heavy chain variable region and a light chain variable region selected from any one of the following: (a) the heavy chain variable region comprising an amino acid sequence having at least 80% identity to a sequence set forth in SEQ ID NO: 87, and the light chain variable region comprising an amino acid sequence having at least 80% identity to a sequence set forth in SEQ ID NO: 88; (b) the heavy chain variable region comprising an amino acid sequence set forth in SEQ ID NO: 87, and the light chain variable region comprising an amino acid sequence set forth in SEQ ID NO: 88; (c) the heavy chain variable region comprising an amino acid sequence set forth in SEQ ID NO: 81, and the light chain variable region comprising an amino acid sequence set forth in SEQ ID NO: 82; or (d) the heavy chain variable region comprising an amino acid sequence set forth in SEQ ID NO: 79, and the light chain variable region comprising an amino acid sequence set forth in SEQ ID NO: 80. In some specific embodiments, the anti-CD47 antibody comprises a heavy chain set forth in SEQ ID NO: 181 and a light chain set forth in SEQ ID NO: 183, or the anti-CD47 antibody comprises a heavy chain set forth in SEQ ID NO: 185 and a light chain set forth in SEQ ID NO: 187. In some specific embodiments, the anti-CD47 antibody is hz14A9-2.3.

In some embodiments, the second therapeutic agent is selected from the group consisting of a hypomethylating agent, an anti-CD20 antibody, an immunomodulator, a proteasome inhibitor, and the like. In some embodiments, the second therapeutic agent is a hypomethylating agent (e.g., azacitidine, etc.). In some embodiments, the second therapeutic agent is an anti-CD20 antibody (e.g., rituximab, etc.). In some embodiments, the second therapeutic agent is an immunomodulator (e.g., bortezomib, etc.) or/and a proteasome inhibitor (e.g., lenalidomide, etc.).

In some embodiments, the hematologic tumor is newly diagnosed, recurrent, or refractory. In some embodiments, the hematologic tumor is recurrent or/and refractory.

In some embodiments, the hematologic tumor is leukemia, lymphoma, myelodysplastic syndrome, or myeloma.

In some embodiments, the hematologic tumor is multiple myeloma, acute lymphoblastic leukemia, acute myeloid leukemia, chronic myeloid leukemia, rare types of leukemia, myelodysplastic syndrome, classical Hodgkin's lymphoma, nodular lymphocyte-predominant Hodgkin's lymphoma, diffuse large B-cell lymphoma, follicular lymphoma, mantle cell lymphoma, marginal zone lymphoma, peripheral T-cell lymphoma, extranodal NK/T-cell lymphoma, extranodal NK/T-cell lymphoma, nasal type, Burkitt lymphoma, chronic lymphocytic leukemia, primary central nerves system lymphoma, lymphoblastic lymphoma, AIDS-related B-cell lymphoma, chronic lymphocytic leukemia, small lymphocytic lymphoma, chronic lymphocytic leukemia/small lymphocytic lymphoma, cutaneous B-cell lymphoma, primary mediastinal large B-cell lymphoma, unclassifiable B-cell lymphoma, Waldenström's macroglobulinemia, or light chain amyloidosis.

The present disclosure also provides a pharmaceutical combination comprising the anti-CD47 antibody described above and the second therapeutic agent described above.

In some embodiments, the pharmaceutical combination is a non-fixed combination. The anti-CD47 antibody and the second therapeutic agent in the non-fixed combination are each in the form of a pharmaceutical composition.

In some embodiments, the pharmaceutical combination is for use in treating the hematologic tumor described above herein. In some embodiments, provided is a pharmaceutical combination for treating the hematologic tumor described above herein, comprising the anti-CD47 antibody described above and the second therapeutic agent described above.

In some embodiments, the present disclosure provides a kit comprising the pharmaceutical combination. The kit may include instructions directing administration of the pharmaceutical combination to a subject with cancer. The kit may also include other materials as may be required from a commercial and user standpoint, for example, other buffers, diluents, needles, syringes, and the like.

*Combination of Anti-CD47 Antibody and Hypomethylating Agent and Use*

In some embodiments, the second therapeutic agent described herein is a hypomethylating agent. Thus, in some embodiments, provided is use of an anti-CD47 antibody and a hypomethylating agent for preparing a medicament for treating a hematologic tumor in a subject. In some embodiments, provided is a method for treating a hematologic tumor in a subject, comprising administering to the subject a therapeutically effective amount of an anti-CD47 antibody and a hypomethylating agent.

In some embodiments, the hypomethylating agent is azacitadine (5-azacytidine), decitabine (5-azadeoxycytidine), guadecitabine (SGI-110), zebularine, or procaine. In some specific embodiments, the hypomethylating agent is azacitadine.

Azacitadine is a cytosine analog, which is an inhibitor of nucleoside metabolism, with the chemical name of 4-amino-1-$\beta$-D-ribofuranosyl-1,3,5-triazin-2($1H$)-one and the molecular formula of $C_8H_{12}N_4O_5$. One example of a marketed drug is the drug VIDAZA®, with azacitadine as the active ingredient. Another example of a marketed drug is the generic drug 维首®, with azacitadine as the active ingredient.

In some embodiments, the anti-CD47 antibody is selected from the group consisting of those described in the section of *"Anti-CD47 Antibody"*. In some specific embodiments, the anti-CD47 antibody comprises: a heavy chain variable region comprising: HCDR1 comprising an amino acid sequence set forth in SEQ ID NO: 5, HCDR2 comprising an amino acid sequence set forth in SEQ ID NO: 10, and HCDR3 comprising an amino acid sequence set forth in SEQ ID NO: 15; and a light chain variable region comprising: LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30. In some specific embodiments, the anti-CD47 antibody comprises a heavy chain variable region and a light chain variable region selected from any one of the following: (a) the heavy chain variable region comprising an amino acid sequence having at least 80% identity to a sequence set forth in SEQ ID NO: 87, and the light chain variable region comprising an amino acid sequence having at least 80% identity to a sequence set forth in SEQ ID NO: 88; (b) the heavy chain variable region comprising an amino acid sequence set forth in SEQ ID NO: 87, and the light chain variable region comprising an amino acid sequence set forth in SEQ ID NO: 88; (c) the heavy chain variable region comprising an amino acid sequence set forth in SEQ ID NO: 81, and the light chain variable region comprising an amino acid sequence set forth in SEQ ID NO: 82; or (d) the heavy chain variable region comprising an amino acid sequence set forth in SEQ ID NO: 79, and the light chain variable region comprising an amino acid sequence set forth in SEQ ID NO: 80. In some specific embodiments, the anti-CD47 antibody comprises a heavy chain set forth in SEQ ID NO: 181 and a light chain set forth in SEQ ID NO: 183, or the anti-CD47 antibody comprises a heavy chain set forth in SEQ ID NO: 185 and a light chain set forth in SEQ ID NO: 187. In some specific embodiments, the anti-CD47 antibody is hz14A9-2.3. In some embodiments, in these specific embodiments of the anti-CD47 antibody, the hypomethylating agent is azacitadine.

In some embodiments, the hematologic tumor is leukemia, lymphoma, myelodysplastic syndrome, or myeloma. In some embodiments, the leukemia is acute leukemia (AL) or chronic leukemia (CL). In some embodiments, the leukemia is acute lymphoblastic leukemia (ALL), acute myeloid leukemia (AML), chronic myeloid leukemia (CML), chronic lymphocytic leukemia (CLL), or rare types of leukemia.

In some embodiments, the hematologic tumor is acute myeloid leukemia.

In some embodiments, the acute myeloid leukemia is recurrent or/and refractory AML.

In some embodiments, the acute myeloid leukemia includes, but is not limited to, various subtypes. Generally, the acute myeloid leukemia can be divided into different subtypes according to different classification criteria or systems. For example, in some embodiments, the acute myeloid leukemia is a subtype under the FAB criteria or under the WHO criteria. For example, in some embodiments, the acute myeloid leukemia is M0 (acute minimally differentiated leukemia), M1 (acute undifferentiated leukemia), M2 (acute partially differentiated myelocytic leukemia), M3 (acute promyelocytic leukemia), M4 (acute myelomonocytic leukemia), M5 (acute monocytic leukemia), M6 (acute erythroleukemia), or M7 (acute megakaryocytic leukemia). For example, in some embodiments, the acute myeloid leukemia is AML with at least one genetic abnormality, AML with multilineage dysplasia, therapy-related AML, undifferentiated AML, AML with minimal maturation, AML with maturation, acute myelomonocytic leukemia, acute monocytic leukemia, acute erythroleukemia, acute megakaryoblastic leukemia, acute basophilic leukemia, acute panmyelosis with myelofibrosis, or myeloid sarcoma.

In some embodiments, the at least one genetic abnormality is selected from the group consisting of a translocation t(8;21)(q22;q22), an inversion inv(16)(p13;q22), a translocation t(16;16)(p13;q22), a translocation t(15;17)(q22;q12), a *PMLIPARα* fusion, an *AML7/ETO* fusion, a *CBFBb/YH11* fusion, an *MLL* rearrangement, a *BCR/ABL1* fusion, a *C-KIT* mutation, an *FLT3* mutation, an *NPM1* mutation, a *CEBPA* mutation, a *TP53* mutation, an *RUNX1(AML1)* mutation, an *ASXLI* mutation, an *IDH1* mutation, an *IDH2* mutation, a *DNMT3α* mutation, a *TET2* mutation, an *SF3B1* mutation, a *U2AF1* mutation, an *SRSF2* mutation, a *ZRSR2* mutation, an *EZH2* mutation, a *BCOR* mutation, an *STAG2* mutation, a *CALR* mutation, an *MPL* mutation, a *CSF3R* mutation, a *CBL* mutation, an *SETBP1* mutation, an *SF3B1* mutation, a *U2AF1* mutation, a *WT1* mutation, a *CSF3R* mutation, a *JAK1* mutation, a *JAK2* mutation, a *JAK3* mutation, a *BRAF* mutation, a *TET2* mutation, a *KRAS* mutation, an *NRAS* mutation, and a *PTPN11* mutation.

In some other embodiments, the hematologic tumor is myelodysplastic syndrome (MDS). In some embodiments, the MDS is recurrent or/and refractory MDS.

In some embodiments, the MDS is MDS with single lineage dysplasia (MDS-SLD), MDS with multiple lineage dysplasia (MDS-MLD), MDS with ring sideroblasts (MDS-RS), MDS with excess blasts (MDS-EB), or MDS, unclassifiable (MDS-U).

In some embodiments, the MDS is low-risk, intermediate-risk, or high-risk MDS.

In some embodiments, the MDS has genetic abnormalities, including those selected from the group consisting of, for example, a chromosomal abnormality of +8, -7/del(7q), del(20q), -5/del(5q), or -Y, and for example, a gene mutation of *TP53* mutation, *TET2* mutation, *DNMT3A* mutation, *IDH1/2* mutation, *EZH2* mutation, *ASXLI* mutation, *SRSF2* mutation, *RUNX1* mutation, *U2AF1* mutation, *SETBP1* mutation, or *SF3B1* mutation, and the like.

The present disclosure also provides a pharmaceutical combination comprising the anti-CD47 antibody described above and the hypomethylating agent described above.

In some embodiments, the pharmaceutical combination is a non-fixed combination. The anti-CD47 antibody and the hypomethylating agent in the non-fixed combination are each in the form of a pharmaceutical composition.

In some embodiments, the pharmaceutical combination is for use in treating the hematologic tumor described above herein. In some embodiments, provided is a pharmaceutical combination for treating the hematologic tumor described above herein, comprising: the anti-CD47 antibody described above and the hypomethylating agent described above.

In some embodiments, the present disclosure provides a kit comprising the pharmaceutical combination.

### *Combination of Anti-CD47 Antibody and Anti-CD20 Antibody and Use*

In some embodiments, the second therapeutic agent is an anti-CD20 antibody. Thus, in some embodiments, provided is use of the anti-CD47 antibody and the anti-CD20 antibody for preparing a medicament for treating a hematologic tumor in a subject.

In some embodiments, provided is a method for treating a hematologic tumor in a subject, comprising administering to the subject a therapeutically effective amount of the anti-CD47 antibody and the anti-CD20 antibody.

In some embodiments, the anti-CD20 antibody comprises obinutuzumab, ofatumumab, ocrelizumab, ibritumomab, tositumomab, rituximab, or ublituximab. In some embodiments, the anti-CD20 antibody comprises HCDR1, HCDR2, HCDR3, LCDR1, LCDR2, and LCDR3 of rituximab. In some embodiments, the anti-CD20 antibody comprises a heavy chain variable region and a light chain variable region of rituximab. In some specific embodiments, the anti-CD20 antibody is rituximab.

Rituximab is an anti-CD20 monoclonal antibody. One example of a marketed drug is the drug Rituxan®, with rituximab as the active ingredient. Another example of a marketed drug is the rituximab biosimilar drug RUXIENCE®.

In some embodiments, the anti-CD47 antibody is selected from the group consisting of those described in the section of *"Anti-CD47 Antibody"*. In some specific embodiments, the anti-CD47 antibody comprises: a heavy chain variable region comprising: HCDR1 comprising an amino acid sequence set forth in SEQ ID NO: 5, HCDR2 comprising an amino acid sequence set forth in SEQ ID NO: 10, and HCDR3 comprising an amino acid sequence set forth in SEQ ID NO: 15; and a light chain variable region comprising: LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30. In some specific embodiments, the anti-CD47 antibody comprises a heavy chain variable region and a light chain variable region selected from any one of the following: (a) the heavy chain variable region comprising an amino acid sequence having at least 80% identity to a sequence set forth in SEQ ID NO: 87, and the light chain variable region comprising an amino acid sequence having at least 80% identity to a sequence set forth in SEQ ID NO: 88; (b) the heavy chain variable region comprising an amino acid sequence set forth in SEQ ID NO: 87, and the light chain variable region comprising an amino acid sequence set forth in SEQ ID NO: 88; (c) the heavy chain variable region comprising an amino acid sequence set forth in SEQ ID NO: 81, and the light chain variable region comprising an amino acid sequence set forth in SEQ ID NO: 82; or (d) the heavy chain variable region comprising an amino acid sequence set forth in SEQ ID NO: 79, and the light chain variable region comprising an amino acid sequence set forth in SEQ ID NO: 80. In some specific embodiments, the anti-CD47 antibody comprises a heavy chain set forth in SEQ ID NO: 181 and a light chain set forth in SEQ ID NO: 183, or the anti-CD47 antibody comprises a heavy chain set forth in SEQ ID NO: 185 and a light chain set forth in SEQ ID NO: 187. In some specific embodiments, the anti-CD47 antibody is hz14A9-2.3. In some embodiments, in these specific embodiments of the anti-CD47 antibody, the anti-CD20 antibody is rituximab.

In some embodiments, the hematologic tumor is leukemia, lymphoma, myelodysplastic syndrome, or myeloma. In some specific embodiments, the hematologic tumor is lymphoma.

In some embodiments, the lymphoma is B cell lymphoma, T cell lymphoma, or NK/T cell lymphoma.

In some embodiments, the lymphoma is indolent lymphoma, aggressive lymphoma, or highly aggressive lymphoma.

In some embodiments, the lymphoma is Hodgkin's lymphoma (HL) or non-Hodgkin's lymphoma (NHL).

In some embodiments, the lymphoma is classical Hodgkin's lymphoma (CHL), nodular lymphocyte-predominant Hodgkin's lymphoma (NLPHL), diffuse large B-cell lymphoma (DLBCL), follicular lymphoma (FL), mantle cell lymphoma (MCL), marginal zone lymphoma (MZL), peripheral T-cell lymphoma (PTCL), extranodal NK/T-cell lymphoma, extranodal NK/T-cell lymphoma, nasal type (NKTCL), Burkitt lymphoma (BL), chronic lymphocytic leukemia (CLL), primary central nerves system lymphoma (PCNSL), lymphoblastic lymphoma, AIDS-related B-cell lymphoma, chronic lymphocytic leukemia (CLL), small lymphocytic lymphoma (SLL), chronic lymphocytic leukemia/small lymphocytic lymphoma (CLL/SLL), cutaneous B-cell lymphoma, primary mediastinal large B-cell lymphoma (PMBL), or unclassifiable B-cell lymphoma.

In some embodiments, the lymphoma is recurrent or refractory. In some embodiments, the lymphoma is recurrent or/and refractory Hodgkin's lymphoma, for example, including, but not limited to, recurrent or/and refractory classical Hodgkin's lymphoma or nodular lymphocyte-predominant Hodgkin's lymphoma. In some embodiments, the lymphoma is recurrent or/and refractory non-Hodgkin's lymphoma, for example, including, but not limited to, recurrent or refractory diffuse large B-cell lymphoma, follicular lymphoma, marginal zone lymphoma, mantle cell lymphoma, peripheral T-cell lymphoma, extranodal NK/T-cell lymphoma, chronic lymphocytic leukemia (CLL), and the like.

In some embodiments, the subject of the lymphoma is CD20-positive (CD20$^+$).

The present disclosure also provides a pharmaceutical combination comprising the anti-CD47 antibody described above and the anti-CD20 antibody described above.

In some embodiments, the pharmaceutical combination is a non-fixed combination. The anti-CD47 antibody and the anti-CD20 antibody in the non-fixed combination are each in the form of a pharmaceutical composition.

In some embodiments, the pharmaceutical combination is for use in treating the hematologic tumor described above herein. In some embodiments, provided is a pharmaceutical combination for treating the hematologic tumor described above herein, comprising: the anti-CD47 antibody described above and the anti-CD20 antibody described above.

In some embodiments, the present disclosure provides a kit comprising the pharmaceutical combination.

### Anti-CD47 Antibody Alone or in Combination with Proteasome Inhibitor or/and Immunosuppressant and Use

In some embodiments, the second therapeutic agent is a proteasome inhibitor or/and an immunosuppressant. Thus, in some embodiments, provided is use of an anti-CD47 antibody and a proteasome inhibitor or/and an immunosuppressant for preparing a medicament for treating a hematologic tumor in a subject.

In some embodiments, provided is a method for treating a hematologic tumor in a subject, comprising administering to the subject a therapeutically effective amount of an anti-CD47 antibody and a proteasome inhibitor or/and an immunosuppressant.

In some embodiments, the proteasome inhibitor is bortezomib, carfilzomib, or ixazomib. In some specific embodiments, the proteasome inhibitor is bortezomib. In some specific embodiments, the proteasome inhibitor is carfilzomib.

In some embodiments, the immunomodulator is lenalidomide, thalidomide, or pomalidomide. In some embodiments, the immunomodulator is lenalidomide. In some embodiments, the immunomodulator is pomalidomide.

In some embodiments, the second therapeutic agent is a combination of a proteasome inhibitor and an immunomodulator. In some specific embodiments, the second therapeutic agent is bortezomib and lenalidomide. In some specific embodiments, the second therapeutic agent is bortezomib and pomalidomide.

Bortezomib is a dipeptidyl borate analog, which is a proteasome inhibitor with the chemical name of [(1R)-3-methyl-1-[[(2S)-1-oxo-3-phenyl-2-[(pyrazinecarboxyl)amino]propyl]amino]butyl]boronic acid and the molecular formula of $C_{19}H_{25}BN_4O_4$. One example of a marketed drug is the drug Velcade® with bortezomib as the active ingredient. Another example of a marketed drug is the generic drug 千平® with bortezomib as the active ingredient.

Lenalidomide is an immunomodulator, with the chemical name of 3-(4'-atnino-1-oxo-1,3-dihydro-2$H$-isoindol-2-yl)piperidine-2,6-dione and the molecular formula of $C_{13}H_{13}N_3O_3$. One example of a marketed drug is the drug Revlimid® with lenalidomide as the active ingredient. Another example of a marketed drug is the generic drug 安显® with lenalidomide as the active ingredient.

In some embodiments, the anti-CD47 antibody is selected from the group consisting of those described in the section of *"Anti-CD47 Antibody".* In some specific embodiments, the anti-CD47 antibody comprises: a heavy chain variable region comprising: HCDR1 comprising an amino acid sequence set forth in SEQ ID NO: 5, HCDR2 comprising an amino acid sequence set forth in SEQ ID NO: 10, and HCDR3 comprising an amino acid sequence set forth in SEQ ID NO: 15; and a light chain variable region comprising: LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30. In some specific embodiments, the anti-CD47 antibody comprises a heavy chain variable region and a light chain variable region selected from any one of the following: (a) the heavy chain variable region comprising an amino acid sequence having at least 80% identity to a sequence set forth in SEQ ID NO: 87, and the light chain variable region comprising an amino acid sequence having at least 80% identity to a sequence set forth in SEQ ID NO: 88; (b) the heavy chain variable region comprising an amino acid sequence set forth in SEQ ID NO: 87, and the light chain variable region comprising an amino acid sequence set forth in SEQ ID NO: 88; (c) the heavy chain variable region comprising an amino acid sequence set forth in SEQ ID NO: 81, and the light chain variable region comprising an amino acid sequence set forth in SEQ ID NO: 82; or (d) the heavy chain variable region comprising an amino acid sequence set forth in SEQ ID NO: 79, and the light chain variable region comprising an amino acid sequence set forth in SEQ ID NO: 80. In some specific embodiments, the anti-CD47 antibody comprises a heavy chain set forth in SEQ ID NO: 181 and a light chain set forth in SEQ ID NO: 183, or the anti-CD47 antibody comprises a heavy chain set forth in SEQ ID NO: 185 and a light chain set forth in SEQ ID NO: 187. In some specific embodiments, the anti-CD47 antibody is hz14A9-2.3. In some embodiments, in these specific embodiments of the anti-CD47 antibody, the second therapeutic agent is bortezomib or lenalidomide, or a combination of both.

In some embodiments, the hematologic tumor is leukemia, lymphoma, myelodysplastic syndrome, or myeloma. In some embodiments, the hematologic tumor is multiple myeloma.

In some embodiments, the multiple myeloma is smoldering myeloma or active multiple myeloma.

In some embodiments, the multiple myeloma is low-risk, standard-risk, or high-risk multiple myeloma. Subjects with high-risk multiple myeloma are known to relapse early and have a poor prognosis and outcome. A subject may be classified as having high-risk multiple myeloma if the subject has one or more of the following cytogenetic abnormalities: t(4;14)(p16;q32), t(14;16)(q32;q23), de117p, 1qAmp, t(4;14)(p16;q32) and t(14;16)(q32;q23), t(4;14)(p16;q32) and de117p, t(14;16)(q32;q23) and de117p, or t(4;14)(p16;q32), (14;16)(q32;q23), and de117p. In some embodiments, a subject with high-risk multiple myeloma has one or more chromosomal abnormalities including: t(4;14)(p16;q32), t(14;16)(q32;q23), de117p, 1qAmp, t(4;14)(p16;q32) and t(14;16)(q32;q23), t(4;14)(p16;q32) and de117p, t(14;16)(q32;q23) and de117p, or t(4;14)(p16;q32), t(14;16)(q32;q23) and de117p, or any combination thereof.

In some embodiments, the multiple myeloma is newly diagnosed multiple myeloma.

In some embodiments, the multiple myeloma is recurrent or/and refractory multiple myeloma.

The present disclosure also provides a pharmaceutical combination comprising the anti-CD47 antibody and the second therapeutic agent described above, wherein the second therapeutic agent comprises the proteasome inhibitor described above or/and the immunosuppressant described above.

In some embodiments, the pharmaceutical combination is a non-fixed combination. The anti-CD47 antibody and the proteasome inhibitor or/and the immunosuppressant in the non-fixed combination are each in the form of a pharmaceutical composition.

In some embodiments, the pharmaceutical combination is for use in treating the hematologic tumor described above herein. In some embodiments, provided is a pharmaceutical combination for treating the hematologic tumor described above herein, comprising: the anti-CD47 antibody and the second therapeutic agent described above, wherein the second therapeutic agent comprises the proteasome inhibitor or/and the immunosuppressant described above.

In some embodiments, the present disclosure provides a kit comprising the pharmaceutical combination.

In some embodiments, provided is use of the anti-CD47 antibody described above for preparing a medicament for treating the myeloma (e.g., multiple myeloma) described above in a subject. In some embodiments, provided is a method for treating the myeloma (e.g., multiple myeloma) described above in a subject, comprising administering to the subject a therapeutically effective amount of the anti-CD47 antibody described above.

*Method of Administration*

The following exemplary methods of administration may be employed when the anti-CD47 antibody is administered alone or in combination with other second therapeutic agents described above:

In some embodiments, the anti-CD47 antibody is administered in a therapeutic dose of 0.5 mg/kg to 70 mg/kg, e.g., 0.5 mg/kg to 40 mg/kg, 15 mg/kg to 40 mg/kg, 15 mg/kg to 30 mg/kg, 25 mg/kg to 35 mg/kg, or 15 mg/kg to 35 mg/kg according to the body weight of the patient. Suitable doses include, for example, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, 25 mg/kg, 26 mg/kg, 27 mg/kg, 28 mg/kg, 29 mg/kg, 30 mg/kg, 31 mg/kg, 32 mg/kg, 33 mg/kg, 34 mg/kg, 35 mg/kg, 36 mg/kg, 37 mg/kg, 38 mg/kg, 39 mg/kg, 40 mg/kg, 41 mg/kg, 42 mg/kg, 43 mg/kg, 44 mg/kg, 45 mg/kg, 50 mg/kg, 55 mg/kg, 60 mg/kg, 65 mg/kg, or 70 mg/kg.

In some embodiments, when administered to a subject with cancer in a therapeutic dose, the anti-CD47 antibody may also be administered in a fixed dose. In some embodiments, the anti-CD47 antibody is administered in a fixed dose of 60 mg to 3000 mg, e.g., 60 mg to 2000 mg, 600 mg to 1800 mg, or 1000 mg to 1800 mg. Suitable fixed doses include, for example, 60 mg, 100 mg, 180 mg, 360 mg, 500 mg, 600 mg, 700 mg, 800 mg, 900 mg, 1000 mg, 1100 mg, 1200 mg, 1300 mg, 1400 mg, 1500 mg, 1600 mg, 1700 mg, 1800 mg, 1900 mg, 2000 mg, 2500 mg, or 3000 mg.

In some embodiments, the anti-CD47 antibody is administered in one, two, or more therapeutic doses every 3-21 days, e.g., every 3-14 days or 7-14 days. Suitable administration frequencies are, for example, administration of a therapeutic dose of the anti-CD47 antibody twice a week, once a week (q1w), once every 2 weeks (q2w), or once every 3 weeks (q3w).

In some embodiments, the anti-CD47 antibody can typically be administered at a therapeutic dose between 1 and 6 times (e.g., 1, 2, 3, 4, 5, or 6 times) to treat cancer (such as liver cancer), and can be administered at a therapeutic dose of 7, 8, 9, 10, 11, 12, 13, 14, or more times.

In some embodiments, a therapeutic dose of the anti-CD47 antibody is administered to a subject with cancer in one treatment cycle of every 2-6 weeks, e.g., one treatment cycle of every 2 weeks (14 days), every 3 weeks (21 days), every 4 weeks (28 days), every 5 weeks (35 days), or every 6 weeks (42 days).

In some embodiments, it is permissible to administer the anti-CD47 antibody in a dose escalation manner to achieve the therapeutic dose.

In some embodiments, it is permissible to administer a sub-therapeutic dose of the anti-CD47 antibody that is less than the therapeutic dose prior to administration of the therapeutic dose of the anti-CD47 antibody.

The anti-CD47 antibody can be administered by a variety of routes, in one embodiment, intravenously.

Regimens such as administration interval, administration dose, and route of administration for the second therapeutic agent may be made in accordance with the properties of each therapeutic agent itself. For example, rituximab may be administered intravenously, azacitadine may be administered by subcutaneous injection, lenalidomide may be administered orally, and bortezomib may be administered intravenously.

In some embodiments, the anti-CD47 antibody may be administered simultaneously, sequentially, or at an interval when used in combination with a second therapeutic agent.

### Anti-CD47 Antibody

Some exemplary anti-CD47 antibodies suitable for use in any of the uses, methods, or pharmaceutical combinations described above are provided below.

In certain embodiments, in addition to binding to human CD47 or/and cynomolgus monkey CD47, the anti-CD47 antibody provided has a number of other desired characteristics for a targeted therapy. Specifically, these desired characteristics may be, for example, at least one of the following: binding to CD47 with a $K_D$ value of 1.53E-08 or less; blocking binding of CD47 to SIRPα; promoting macrophage-mediated phagocytosis of CD47-expressing cells; not significantly inducing apoptosis of CD4$^+$ T cells; not causing substantial hematocytopenia, anemia or red cell agglutination; and the antigen-binding polypeptide or the antigen-binding portion having a melting temperature T of $\geq 62$ °C and an aggregation temperature Tagg of $\geq 61$ °C. In some embodiments, the anti-CD47 antibody has a better therapeutic effect; in some embodiments, the anti-CD47 antibody has reduced side effects. In some embodiments, the anti-CD47 antibody is a chimeric, humanized, or human antibody. In a specific embodiment, the anti-CD47 antibody is humanized.

In some embodiments, the anti-CD47 antibody is of an IgG1, IgG2, IgG3, or IgG4 subclass. In some embodiments, the anti-CD47 antibody is of an IgG4 subclass. In some embodiments, the anti-CD47 antibody is of an IgG4 subclass and contains an amino acid substitution of S228P according to EU numbering.

In some embodiments, the anti-CD47 antibody comprises:

a heavy chain variable region comprising: HCDR1 comprising an amino acid sequence set forth in SEQ ID NO: 1, HCDR2 comprising an amino acid sequence set forth in SEQ ID NO: 6, and HCDR3 comprising an amino acid sequence set forth in SEQ ID NO: 11; and
a light chain variable region comprising: LCDR1 comprising an amino acid sequence set forth in SEQ ID NO: 16, LCDR2 comprising an amino acid sequence set forth in SEQ ID NO: 21, and LCDR3 comprising an amino acid sequence set forth in SEQ ID NO: 26.

In some embodiments, the anti-CD47 antibody comprises:

a heavy chain variable region comprising: HCDR1 comprising an amino acid sequence set forth in SEQ ID NO: 2, HCDR2 comprising an amino acid sequence set forth in SEQ ID NO: 7, and HCDR3 comprising an amino acid sequence set forth in SEQ ID NO: 12; and
a light chain variable region comprising: LCDR1 comprising an amino acid sequence set forth in SEQ ID NO: 17, LCDR2 comprising an amino acid sequence set forth in SEQ ID NO: 22, and LCDR3 comprising an amino acid sequence set forth in SEQ ID NO: 27.

In some embodiments, the anti-CD47 antibody comprises:

a heavy chain variable region comprising: HCDR1 comprising an amino acid sequence set forth in SEQ ID NO: 3, HCDR2 comprising an amino acid sequence set forth in SEQ ID NO: 8, and HCDR3 comprising an amino acid sequence set forth in SEQ ID NO: 13; and
a light chain variable region comprising: LCDR1 comprising an amino acid sequence set forth in SEQ ID NO: 18, LCDR2 comprising an amino acid sequence set forth in SEQ ID NO: 23, and LCDR3 comprising an amino acid sequence set forth in SEQ ID NO: 28.

In some embodiments, the anti-CD47 antibody comprises:

a heavy chain variable region comprising: HCDR1 comprising an amino acid sequence set forth in SEQ ID NO: 4, HCDR2 comprising an amino acid sequence set forth in SEQ ID NO: 9, and HCDR3 comprising an amino acid sequence set forth in SEQ ID NO: 14; and
a light chain variable region comprising: LCDR1 comprising an amino acid sequence set forth in SEQ ID NO: 19, LCDR2 comprising an amino acid sequence set forth in SEQ ID NO: 24, and LCDR3 comprising an amino acid sequence set forth in SEQ ID NO: 32.

In some embodiments, the anti-CD47 antibody comprises:

a heavy chain variable region comprising: HCDR1 comprising an amino acid sequence set forth in SEQ ID NO: 5, HCDR2 comprising an amino acid sequence set forth in SEQ ID NO: 10, and HCDR3 comprising an amino acid sequence set forth in SEQ ID NO: 15; and
a light chain variable region comprising: LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30.

In some embodiments, the anti-CD47 antibody comprises a heavy chain variable region and a light chain variable region, wherein the heavy chain variable region comprises HCDR1, HCDR2, and HCDR3 of a variable region sequence set forth in SEQ ID NO: 33, 35, 37, 39, 41, 83, 85 or 87, and the light chain variable region comprises LCDR1, LCDR2, and LCDR3 of a variable region sequence set forth in SEQ ID NO: 34, 36, 38, 40, 42, 84, 86 or 88. In some specific embodiments, the heavy chain variable region of the anti-CD47 antibody comprises HCDR1, HCDR2, and HCDR3 of a variable region sequence set forth in SEQ ID NO: 83, and the light chain variable region of the anti-CD47 antibody comprises LCDR1, LCDR2, and LCDR3 of a variable region sequence set forth in SEQ ID NO: 84. In some specific embodiments, the heavy chain variable region of the anti-CD47 antibody comprises HCDR1, HCDR2, and HCDR3 of a variable region sequence set forth in SEQ ID NO: 85, and the light chain variable region of the anti-CD47 antibody comprises LCDR1, LCDR2, and LCDR3 of a variable region sequence set forth in SEQ ID NO: 86. In some specific embodiments, the heavy chain variable region of the anti-CD47 antibody comprises HCDR1, HCDR2, and HCDR3 of a variable region sequence set forth in SEQ ID NO: 87, and the light chain variable region of the anti-CD47 antibody comprises LCDR1, LCDR2, and LCDR3 of a variable region sequence set forth in SEQ ID NO: 88.

In some embodiments, the heavy chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 33, 35, 37, 39, 41, 83, 85, or 87.

In some embodiments, the light chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 34, 36, 38, 40, 42, 84, 86, or 88.

In some embodiments, the heavy chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 39, and the light chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 40. Further, in some of these embodiments, the anti-CD47 antibody further comprises HCDR1 comprising an amino acid sequence set forth in SEQ ID NO: 4, HCDR2 comprising an amino acid sequence set forth in SEQ ID NO: 9, HCDR3 comprising an amino acid sequence set forth in SEQ ID NO: 14, LCDR1 comprising an amino acid sequence set forth in SEQ ID NO: 19, LCDR2 comprising an amino acid sequence set forth in SEQ ID NO: 24, and LCDR3 comprising an amino acid sequence set forth in SEQ ID NO: 32.

In some embodiments, the heavy chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 41, and the light chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 42. Further, in some of these embodiments, the anti-CD47 antibody further comprises HCDR1 comprising an amino acid sequence set forth in SEQ ID NO: 5, HCDR2 comprising an amino acid sequence set forth in SEQ ID NO: 10, HCDR3 comprising an amino acid sequence set forth in SEQ ID NO: 15, LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30.

In some embodiments, the heavy chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 83, and the light chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 84. Further, in some of these embodiments, the anti-CD47 antibody further comprises HCDR1 comprising an amino acid sequence set forth in SEQ ID NO: 4, HCDR2 comprising an amino acid sequence set forth in SEQ ID NO: 9, HCDR3 comprising an amino acid sequence set forth in SEQ ID NO: 14, LCDR1 comprising an amino acid sequence set forth in SEQ ID NO: 19, LCDR2 comprising an amino acid sequence set forth in SEQ ID NO: 24, and LCDR3 comprising an amino acid sequence set forth in SEQ ID NO: 32.

In some embodiments, the heavy chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 87, and the light chain variable region of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 88. Further, in some of these embodiments, the anti-CD47 antibody further comprises HCDR1 comprising an amino acid sequence set forth in SEQ ID NO: 5, HCDR2 comprising an amino acid sequence set forth in SEQ ID NO: 10, HCDR3 comprising an amino acid sequence set forth in SEQ ID NO: 15, LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30.

In some specific embodiments, the heavy chain variable region of the anti-CD47 antibody comprises an amino acid sequence set forth in SEQ ID NO: 39, and the light chain variable region of the anti-CD47 antibody comprises an amino acid sequence set forth in SEQ ID NO: 40.

In some specific embodiments, the heavy chain variable region of the anti-CD47 antibody comprises an amino acid sequence set forth in SEQ ID NO: 41, and the light chain variable region of the anti-CD47 antibody comprises an amino acid sequence set forth in SEQ ID NO: 42.

In some specific embodiments, the heavy chain variable region of the anti-CD47 antibody comprises an amino acid sequence set forth in SEQ ID NO: 83, and the light chain variable region of the anti-CD47 antibody comprises an amino acid sequence set forth in SEQ ID NO: 84.

In some specific embodiments, the heavy chain variable region of the anti-CD47 antibody comprises an amino acid sequence set forth in SEQ ID NO: 87, and the light chain variable region of the anti-CD47 antibody comprises an amino acid sequence set forth in SEQ ID NO: 88.

In some specific embodiments, the heavy chain variable region of the anti-CD47 antibody comprises an amino acid sequence set forth in SEQ ID NO: 81, and the light chain variable region of the anti-CD47 antibody comprises an amino acid sequence set forth in SEQ ID NO: 82.

In some specific embodiments, the heavy chain variable region of the anti-CD47 antibody comprises an amino acid sequence set forth in SEQ ID NO: 79, and the light chain variable region of the anti-CD47 antibody comprises an amino acid sequence set forth in SEQ ID NO: 80.

In some other specific embodiments, the heavy chain variable region and the light chain variable region of the anti-CD47 antibody comprise amino acid sequences selected from the group consisting of SEQ ID NOs: 33/34, 35/36, 37/38, 43/44, 45/46, 47/48, 49/50, 51/52, 53/54, 55/56, 57/58, 59/60, 61/62, 63/64, 65/66, 67/68, 69/70, 71/72, 73/74, 75/76, 77/78, and 85/86.

In some embodiments, the anti-CD47 antibody comprises a heavy chain and a light chain. In some embodiments, the heavy chain of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 181, and the light chain of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 183. In one specific embodiment, the anti-CD47 antibody comprises a heavy chain set forth in SEQ ID NO: 181 and a light chain set forth in SEQ ID NO: 183. In some other embodiments, the heavy chain of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 185, and the light chain of the anti-CD47 antibody comprises an amino acid sequence having at least 80%, 85%, 86%, 87%, 88%, 89%, 90%, 91%, 92%, 93%, 94%, 95%, 96%, 97%, 98%, 99%, or 100% identity to a sequence set forth in SEQ ID NO: 187. In one specific embodiment, the anti-CD47 antibody comprises a heavy chain set forth in SEQ ID NO: 185 and a light chain set forth in SEQ ID NO: 187. In some other embodiments, the anti-CD47 antibody may further comprise a heavy chain and a light chain of Xi2H8, Xi16E5, Xi2B2, Xi3F10, Xi14A9, hz3F10-1.1, hz3F10-2.1, hz3F10-3.1, hz3F10-4.1, hz3F10-5.1, hz3F10-6.1, hz3F10-1.2, hz3F10-2.2, hz3F10-3.2, hz3F10-4.2, hz3F10-5.2, hz3F10-6.2, hz16E5-1.1, hz16E5-1.3, hz16E5-1.2, hz16E5-3.2, hz16E5-3.1, or hz16E5-3.3.

Also provided are some exemplary monoclonal antibodies that bind to CD47, including chimeric versions (Xi2B2, Xi2H8, Xi3F10, Xi16E5, and Xi14A9) of 2B2, 2H8, 3F10, 16E5, and 14A9 and humanized variants of 3F10, 16E5, and 14A9. The amino acid sequences of HCDRs (HCDR1, HCDR2, and HCDR3) of the exemplary antibodies that bind to CD47 provided herein are provided in Table S1 below. The amino acid sequences of LCDRs (LCDR1, LCDR2, and LCDR3) are provided in Table S2 below. The amino acid sequences of the variable regions and the full-length heavy and light chains of part of the antibodies are provided in Tables S3 and S4 below.

Table S1: Heavy chain CDR sequences of anti-CD47 antibodies

| Name of antibody | Heavy chain CDR1 | Heavy chain CDR2 | Heavy chain CDR3 |
|---|---|---|---|
| Xi2H8 | GFSLTNYGVH (SEQ ID NO: 1) | IIWAGGSTN (SEQ ID NO: 6) | DDYASMDY (SEQ ID NO: 11) |
| Xi2B2 | GFTFSNYYMS (SEQ ID NO: 2) | YISSGGGSTY (SEQ ID NO: 7) | FAY (SEQ ID NO: 12) |
| X116E5 | GYTFTNYGMN (SEQ ID NO: 3) | WINTNTGEPT (SEQ ID NO: 8) | FSHLRGPMDY (SEQ ID NO: 13) |
| Xi3F10 | GYTFSRYWIE (SEQ ID NO: 4) | EFIPGSDT (SEQ ID NO: 9) | GGLRRMDY (SEQ ID NO: 14) |
| Xi14A9 | GFNIEDDYIE (SEQ ID NO: 5) | RIDPANDKTK (SEQ ID NO: 10) | PGLRRYYSMDY (SEQ ID NO: 15) |
| hz3F10-1.1 / 2.1 / 3.1 / 4.1 / 5.1 / 6.1 / 1.2 / 2.2 / 3.2 / 4.2 / 5.2 / 6.2 | GYTFSRYWIE (SEQ ID NO: 4) | EFIPGSDT (SEQ ID NO: 9) | GGLRRMDY (SEQ ID NO: 14) |
| hz16E5-1.1 / 1.3 / 1.2 / 3.2 / 3.1 / 3.3 | GYTFTNYGMN (SEQ ID NO: 3) | WINTNTGEPT (SEQ ID NO: 8) | FSHLRGPMDY (SEQ ID NO: 13) |
| hz14A9-2.3 / 2.4 | GFNIEDDYIE (SEQ ID NO: 5) | RIDPANDKTK (SEQ ID NO: 10) | PGLRRYYSMDY (SEQ ID NO: 15) |

Table S2: Light chain CDR sequences of anti-CD47 antibodies

| Name | Light chain CDR1 | Light chain CDR2 | Light chain CDR3 |
|---|---|---|---|
| Xi2H8 | RSSQSLVHSNGNTYLH (SEQ ID NO: 16) | KVSNRFS (SEQ ID NO: 21) | SQSTHVPYT (SEQ ID NO: 26) |
| Xi2B2 | KSSQSLLDSDGKTYLN (SEQ ID NO: 17) | LVSKLDS (SEQ ID NO: 22) | GTHFPLT (SEQ ID NO: 27) |

(continued)

| Name | Light chain CDR1 | Light chain CDR2 | Light chain CDR3 |
|------|------------------|------------------|------------------|
| Xi16E5 | RSSQSLVHSNGYTYLH (SEQ ID NO: 18) | KVSNRFS (SEQ ID NO: 23) | SQSTHVPPT (SEQ ID NO: 28) |
| Xi3F10 | RASSSVSSTYLH (SEQ ID NO: 19) | TTSTLAS (SEQ ID NO: 24) | QQFSDSTWT (SEQ ID NO: 29) |
| Xi14A9 | KASENVVSYVS (SEQ ID NO: 20) | GASNRYT (SEQ ID NO: 25) | GQSYSYPLT (SEQ ID NO: 30) |
| hz3F10-1.1 / 3.1 / 5.1 / 1.2 / 3.2 / 5.2 | RASSSVSSTYLH (SEQ ID NO: 19) | TTSTLAS (SEQ ID NO: 24) | QQFSDSTWT (SEQ ID NO: 29) |
| hz3F10-2.1 / 4.1 / 6.1 / 2.2 / 4.2 / 6.2 | RASSSVSSTYLH (SEQ ID NO: 19) | TTSTLAS (SEQ ID NO: 24) | QQFSESTWT (SEQ ID NO: 31) |
| hz16E5-1.1 / 1.3 / 1.2 / 3.2 / 3.1 / 3.3 | RSSQSLVHSNGYTYLH (SEQ ID NO: 18) | KVSNRFS (SEQ ID NO: 23) | SQSTHVPPT (SEQ ID NO: 28) |
| hz14A9-2.3 / 2.4 | KASENVVSYVS (SEQ ID NO: 20) | GASNRYT (SEQ ID NO: 25) | GQSYSYPLT (SEQ ID NO: 30) |
| Humanized 3F10 (common) | RASSSVSSTYLH (SEQ ID NO: 19) | TTSTLAS (SEQ ID NO: 24) | QQFSX$_2$STWT, wherein X$_2$ is D or E (SEQ ID NO: 32) |

Table S3: Variable region seauences of part of anti-CD47 antibodies

| Name | Region | Amino acid sequence | SEQ ID NO: |
|------|--------|---------------------|------------|
| hz14A9-2.3 | VH | QVQLVQSGAEVKKPGASVKVSCKVSGFNIEDDYIEWVRQAPGQGLEWMGRIDPANDKTKYAQKFQGRVTMTGDTSTNTVYMELSSLRSEDTAVYYCARPGLRRYYSMDYWGQGTLVTVSS | 79 |
| | VL | DIQMTQSPSSLSASVGDRVTITCKASENVVSYVSWYQQKPGKAPKLLIYGASNRYTGVPSRFIGSGSSTDFTLTISSLQPEDFATYYCGQSYSYPLTFGQGTKLEIK | 80 |
| hz14A9-2.4 | VH | YSMDYWGQGTLVTVSS | 81 |
| | VL | DIQMTQSPSSLSASVGDRVTITCKASENVVSYVSWYQQKPGKAPKLLIYGASNRYTGVPSRFIGSGSSTDFTLTISSLQPEDFATYYCGQSYSYPLTFGQGTKLEIK | 82 |

(continued)

| Name | Region | Amino acid sequence | SEQ ID NO: |
|------|--------|---------------------|------------|
| Humanized 3F10 (common) | VH | QVQLVQSGAEVKKPGSSVKVSCKASGYTFSRYWIE WVRQAPGQGLEWMGEFIPGSDTTNYAQKFQGRVTI TAX$_3$X$_4$STX$_5$TAYMELSSLRSEDTAVYYCARGGLRR MDYWGQGTLVTVSS <br> wherein X$_3$ is D or E, X$_4$ is I or E, and X$_5$ is S or N | 83 |
| | VL | DIQMTQSPSSLSASVGDRVTITCRASSSVSSTYLHW YQQKPGKAPKLX$_6$IYTTSTLASGVPSRFSGSGSGTX$_7$ X$_8$TLTISSLQPEDFATYYCQQFS X$_2$STWTFGQGTKLEIK <br> wherein X$_6$ is L or W, X$_7$ is D or S, X$_8$ is F or Y, and X$_2$ is D or E | 84 |
| Humanized 14A9 (common) | VH | QVQLVQSGAEVKKPGASVKVSCKX$_{15}$SGFNIEDDYI EWVRQAPGQGLEWMGRIDPANDKTKYAQKFQGRV TMTX$_{16}$DTSTX$_{17}$TVYMELSSLRSEDTAVYYC X$_{18}$RPGLRRYYSMDYWGQGTLVTVSS <br> wherein X$_{15}$ is A or V, X$_{16}$ is R or G, X$_{17}$ is S or N, X$_{18}$ is A or T | 87 |
| | VL | DIQMTQSPSSLSASVGDRVTITCKASENVVSYVSWY QQKPGKAPKLLIYGASNRYTGVPSRFX$_{19}$GSGSX$_{20}$T DFTLTISSLQPEDFATYYCGQSYSYPLTFGQGTKLEI K <br> wherein X$_{19}$ is S or I, and X$_{20}$ is S or G | 88 |

Table S4: Sequences of full-length heavy chains and full-length light chains of part of anti-CD47 antibodies

| Name | Region | Amino acid sequence | SEQ ID NO: |
|------|--------|---------------------|------------|
| hz 14A9-2.3 | Heavy chain | QVQLVQSGAEVKKPGASVKVSCKVSGFNIEDDYIEWVRQ APGQGLEWMGRIDPANDKTKYAQKFQGRVTMTGDTSTNT VYMELSSLRSEDTAVYYCARPGLRRYYSMDYWGQGTLVT VSS ASTKGPSVFPLAPCSRSTSESTAALGCLVKDYFPEPVTVSW NSGALTSGVHTFPAVLQSSGLYSLSSVVTVPSSSLGTKTYT CNVDHKPSNTKVDKRVESKYGPPCPPCPAPEFLGGPSVFLF PPKPKDTLMISRTPEVTCVVVDVSQEDPEVQFNWYVDGV EVHNAKTKPREEQFNSTYRVVSVLTVLHQDWLNGKEYKC KVSNKGLPSSIEKTISKAKGQPREPQVYTLPPSQEEMTKNQ VSLTCLVKGFYPSDIAVEWESNGQPENNYKTTPPVLDSDGS FFLYSRLTVDKSRWQEGNVFSCSVMHEALHNHYTQKSLSL SLGK | 181 |
| | Light chain | DIQMTQSPSSLSASVGDRVTITCKASENVVSYVSWYQQKP GKAPKLLIYGASNRYTGVPSRFIGSGSSTDFTLTISSLQPEDF ATYYCGQSYSYPLTFGQGTKLEIK RTVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC | 183 |

(continued)

| Name | Region | Amino acid sequence | SEQ ID NO: |
|---|---|---|---|
| hz 14A9-2.4 | Heavy chain | QVQLVQSGAEVKKPGASVKVSCKVSGFNIEDDYIEWVRQ APGQGLEWMGRIDPANDKTKYAQKFQGRVTMTGDTSTNT VYMELSSLRSEDTAVYYCTRPGLRRYYSMDYWGQGTLVT VSS ASTKGPSVFPLAPCSRSTSESTAALGCLVKDYFPEPVTVSW NSGALTSGVHTFPAVLQSSGLYSLSSVVTVPSSSLGTKTYT CNVDHKPSNTKVDKRVESKYGPPCPPCPAPEFLGGPSVFLF PPKPKDTLMISRTPEVTCVVVDVSQEDPEVQFNWYVDGV EVHNAKTKPREEQFNSTYRVVSVLTVLHQDWLNGKEYKC KVSNKGLPSSIEKTISKAKGQPREPQVYTLPPSQEEMTKNQ VSLTCLVKGFYPSDIAVEWESNGQPENNYKTTPPVLDSDGS FFLYSRLTVDKSRWQEGNVFSCSVMHEALHNHYTQKSLSL SLGK | 185 |
| | Light chain | DIQMTQSPSSLSASVGDRVTITCKASENVVSYVSWYQQKP GKAPKLLIYGASNRYTGVPSRFIGSGSSTDFTLTISSLQPEDF ATYYCGQSYSYPLTFGQGTKLEIK RTVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC | 187 |

In certain specific embodiments, the anti-CD47 antibody is hz14A9-2.3 or hz14A9-2.4.

The anti-CD47 antibody can be prepared using methods well known in the art. For example, a host cell comprising a nucleic acid encoding the anti-CD47 antibody is cultured under conditions suitable for expression of the anti-CD47 antibody, and the anti-CD47 antibody is isolated from the host cell or host cell culture medium. To produce an anti-CD47 antibody, a nucleic acid encoding the antibody is isolated and inserted into one or more vectors for further cloning or/and expression in a host cell. The nucleic acid can be obtained using various methods known in the art, such as gene cloning, gene splicing and chemical synthesis. The isolation can be performed through a step such as centrifugation or affinity chromatography.

Some other exemplary embodiments are also provided herein, but are not limited thereto:

Embodiment 1. Use of an anti-CD47 antibody and a second therapeutic agent for preparing a medicament for treating a hematologic tumor in a subject.

Embodiment 2. A method for treating a hematologic tumor in a subject, comprising administering to the subject a therapeutically effective amount of an anti-CD47 antibody and a second therapeutic agent.

Embodiment 3. A pharmaceutical combination comprising an anti-CD47 antibody and a second therapeutic agent.

Embodiment 4. The pharmaceutical combination, method, or use according to any one of embodiments 1-3, wherein the anti-CD47 antibody comprises:

(i) a heavy chain variable region comprising:

HCDR1 comprising an amino acid sequence set forth in SEQ ID NO: 1, HCDR2 comprising an amino acid sequence set forth in SEQ ID NO: 6, and HCDR3 comprising an amino acid sequence set forth in SEQ ID NO: 11; and

a light chain variable region comprising: LCDR1 comprising an amino acid sequence set forth in SEQ ID NO: 16, LCDR2 comprising an amino acid sequence set forth in SEQ ID NO: 21, and LCDR3 comprising an amino acid sequence set forth in SEQ ID NO: 26;

(ii) a heavy chain variable region comprising:

HCDR1 comprising an amino acid sequence set forth in SEQ ID NO: 2, HCDR2 comprising an amino acid sequence set forth in SEQ ID NO: 7, and HCDR3 comprising an amino acid sequence set forth in SEQ ID NO: 12; and

a light chain variable region comprising: LCDR1 comprising an amino acid sequence set forth in SEQ ID NO: 17, LCDR2 comprising an amino acid sequence set forth in SEQ ID NO: 22, and LCDR3 comprising an amino acid sequence set forth in SEQ ID NO: 27;

(iii) a heavy chain variable region comprising:

HCDR1 comprising an amino acid sequence set forth in SEQ ID NO: 3, HCDR2 comprising an amino acid sequence set forth in SEQ ID NO: 8, and HCDR3 comprising an amino acid sequence set forth in SEQ ID NO: 13; and
a light chain variable region comprising: LCDR1 comprising an amino acid sequence set forth in SEQ ID NO: 18, LCDR2 comprising an amino acid sequence set forth in SEQ ID NO: 23, and LCDR3 comprising an amino acid sequence set forth in SEQ ID NO: 28;

(iv) a heavy chain variable region comprising:

HCDR1 comprising an amino acid sequence set forth in SEQ ID NO: 4, HCDR2 comprising an amino acid sequence set forth in SEQ ID NO: 9, and HCDR3 comprising an amino acid sequence set forth in SEQ ID NO: 14; and
a light chain variable region comprising:
LCDR1 comprising an amino acid sequence set forth in SEQ ID NO: 19, LCDR2 comprising an amino acid sequence set forth in SEQ ID NO: 24, and LCDR3 comprising an amino acid sequence set forth in SEQ ID NO: 32; or

(v) a heavy chain variable region comprising:

HCDR1 comprising an amino acid sequence set forth in SEQ ID NO: 5, HCDR2 comprising an amino acid sequence set forth in SEQ ID NO: 10, and HCDR3 comprising an amino acid sequence set forth in SEQ ID NO: 15; and
a light chain variable region comprising:
LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30.

Embodiment 5. The pharmaceutical combination, method, or use according to any one of embodiments 1-4, wherein the anti-CD47 antibody comprises a heavy chain variable region and a light chain variable region selected from any one of the following:

(a) the heavy chain variable region comprising an amino acid sequence having at least 80% identity to a sequence set forth in SEQ ID NO: 33, and the light chain variable region comprising an amino acid sequence having at least 80% identity to a sequence set forth in SEQ ID NO: 34;
(b) the heavy chain variable region comprising an amino acid sequence having at least 80% identity to a sequence set forth in SEQ ID NO: 35, and the light chain variable region comprising an amino acid sequence having at least 80% identity to a sequence set forth in SEQ ID NO: 36;
(c) the heavy chain variable region comprising an amino acid sequence having at least 80% identity to a sequence set forth in SEQ ID NO: 37, and the light chain variable region comprising an amino acid sequence having at least 80% identity to a sequence set forth in SEQ ID NO: 38;
(d) the heavy chain variable region comprising an amino acid sequence having at least 80% identity to a sequence set forth in SEQ ID NO: 39, and the light chain variable region comprising an amino acid sequence having at least 80% identity to a sequence set forth in SEQ ID NO: 40;
(e) the heavy chain variable region comprising an amino acid sequence having at least 80% identity to a sequence set forth in SEQ ID NO: 41, and the light chain variable region comprising an amino acid sequence having at least 80% identity to a sequence set forth in SEQ ID NO: 42;
(f) the heavy chain variable region comprising an amino acid sequence having at least 80% identity to a sequence set forth in SEQ ID NO: 83, and the light chain variable region comprising an amino acid sequence having at least 80% identity to a sequence set forth in SEQ ID NO: 84;
(g) the heavy chain variable region comprising an amino acid sequence having at least 80% identity to a sequence set forth in SEQ ID NO: 85, and the light chain variable region comprising an amino acid sequence having at least 80% identity to a sequence set forth in SEQ ID NO: 86; and

(h) the heavy chain variable region comprising an amino acid sequence having at least 80% identity to a sequence set forth in SEQ ID NO: 87, and the light chain variable region comprising an amino acid sequence having at least 80% identity to a sequence set forth in SEQ ID NO: 88.

Embodiment 6. The pharmaceutical combination, method, or use according to any one of embodiments 1-5, wherein the anti-CD47 antibody comprises a heavy chain variable region and a light chain variable region selected from any one of the following:

(a) the heavy chain variable region comprising an amino acid sequence set forth in SEQ ID NO: 33, and the light chain variable region comprising an amino acid sequence set forth in SEQ ID NO: 34;
(b) the heavy chain variable region comprising an amino acid sequence set forth in SEQ ID NO: 35, and the light chain variable region comprising an amino acid sequence set forth in SEQ ID NO: 36;
(c) the heavy chain variable region comprising an amino acid sequence set forth in SEQ ID NO: 37, and the light chain variable region comprising an amino acid sequence set forth in SEQ ID NO: 38;
(d) the heavy chain variable region comprising an amino acid sequence set forth in SEQ ID NO: 39, and the light chain variable region comprising an amino acid sequence set forth in SEQ ID NO: 40;
(e) the heavy chain variable region comprising an amino acid sequence set forth in SEQ ID NO: 41, and the light chain variable region comprising an amino acid sequence set forth in SEQ ID NO: 42;
(f) the heavy chain variable region comprising an amino acid sequence set forth in SEQ ID NO: 83, and the light chain variable region comprising an amino acid sequence set forth in SEQ ID NO: 84;
(g) the heavy chain variable region comprising an amino acid sequence set forth in SEQ ID NO: 85, and the light chain variable region comprising an amino acid sequence set forth in SEQ ID NO: 86;
(h) the heavy chain variable region comprising an amino acid sequence set forth in SEQ ID NO: 87, and the light chain variable region comprising an amino acid sequence set forth in SEQ ID NO: 88;
(i) the heavy chain variable region comprising an amino acid sequence set forth in SEQ ID NO: 81, and the light chain variable region comprising an amino acid sequence set forth in SEQ ID NO: 82; or
(j) the heavy chain variable region comprising an amino acid sequence set forth in SEQ ID NO: 79, and the light chain variable region comprising an amino acid sequence set forth in SEQ ID NO: 80.

Embodiment 7. The pharmaceutical combination, method, or use according to any one of embodiments 1-6, wherein the anti-CD47 antibody is selected from any one of the following:

the anti-CD47 antibody comprising a heavy chain set forth in SEQ ID NO: 89 and a light chain set forth in SEQ ID NO: 91;
the anti-CD47 antibody comprising a heavy chain set forth in SEQ ID NO: 93 and a light chain set forth in SEQ ID NO: 95;
the anti-CD47 antibody comprising a heavy chain set forth in SEQ ID NO: 97 and a light chain set forth in SEQ ID NO: 99;
the anti-CD47 antibody comprising a heavy chain set forth in SEQ ID NO: 101 and a light chain set forth in SEQ ID NO: 103;
the anti-CD47 antibody comprising a heavy chain set forth in SEQ ID NO: 105 and a light chain set forth in SEQ ID NO: 107;
the anti-CD47 antibody comprising a heavy chain set forth in SEQ ID NO: 109 and a light chain set forth in SEQ ID NO: 111;
the anti-CD47 antibody comprising a heavy chain set forth in SEQ ID NO: 113 and a light chain set forth in SEQ ID NO: 115;
the anti-CD47 antibody comprising a heavy chain set forth in SEQ ID NO: 117 and a light chain set forth in SEQ ID NO: 119;
the anti-CD47 antibody comprising a heavy chain set forth in SEQ ID NO: 121 and a light chain set forth in SEQ ID NO: 123;
the anti-CD47 antibody comprising a heavy chain set forth in SEQ ID NO: 125 and a light chain set forth in SEQ ID NO: 127;
the anti-CD47 antibody comprising a heavy chain set forth in SEQ ID NO: 129 and a light chain set forth in SEQ ID NO: 131;
the anti-CD47 antibody comprising a heavy chain set forth in SEQ ID NO: 133 and a light chain set forth in SEQ ID NO: 135;
the anti-CD47 antibody comprising a heavy chain set forth in SEQ ID NO: 137 and a light chain set forth in SEQ ID NO: 139;

the anti-CD47 antibody comprising a heavy chain set forth in SEQ ID NO: 141 and a light chain set forth in SEQ ID NO: 143;

the anti-CD47 antibody comprising a heavy chain set forth in SEQ ID NO: 145 and a light chain set forth in SEQ ID NO: 147;

the anti-CD47 antibody comprising a heavy chain set forth in SEQ ID NO: 149 and a light chain set forth in SEQ ID NO: 151;

the anti-CD47 antibody comprising a heavy chain set forth in SEQ ID NO: 153 and a light chain set forth in SEQ ID NO: 155;

the anti-CD47 antibody comprising a heavy chain set forth in SEQ ID NO: 157 and a light chain set forth in SEQ ID NO: 159;

the anti-CD47 antibody comprising a heavy chain set forth in SEQ ID NO: 161 and a light chain set forth in SEQ ID NO: 163;

the anti-CD47 antibody comprising a heavy chain set forth in SEQ ID NO: 165 and a light chain set forth in SEQ ID NO: 167;

the anti-CD47 antibody comprising a heavy chain set forth in SEQ ID NO: 169 and a light chain set forth in SEQ ID NO: 171;

the anti-CD47 antibody comprising a heavy chain set forth in SEQ ID NO: 173 and a light chain set forth in SEQ ID NO: 175;

the anti-CD47 antibody comprising a heavy chain set forth in SEQ ID NO: 177 and a light chain set forth in SEQ ID NO: 179;

the anti-CD47 antibody comprising a heavy chain set forth in SEQ ID NO: 181 and a light chain set forth in SEQ ID NO: 183; or

the anti-CD47 antibody comprising a heavy chain set forth in SEQ ID NO: 185 and a light chain set forth in SEQ ID NO: 187.

Embodiment 8. The pharmaceutical combination, method, or use according to any one of embodiments 1-3, wherein the anti-CD47 antibody comprises:
a heavy chain variable region comprising:

HCDR1 comprising an amino acid sequence set forth in SEQ ID NO: 5, HCDR2 comprising an amino acid sequence set forth in SEQ ID NO: 10, and HCDR3 comprising an amino acid sequence set forth in SEQ ID NO: 15; and
a light chain variable region comprising:
LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30.

Embodiment 9. The pharmaceutical combination, method, or use according to any one of embodiments 1-3 and 8, wherein the anti-CD47 antibody comprises a heavy chain variable region and a light chain variable region selected from any one of the following:

(a) the heavy chain variable region comprising an amino acid sequence having at least 80% identity to a sequence set forth in SEQ ID NO: 87, and the light chain variable region comprising an amino acid sequence having at least 80% identity to a sequence set forth in SEQ ID NO: 88;

(b) the heavy chain variable region comprising an amino acid sequence set forth in SEQ ID NO: 87, and the light chain variable region comprising an amino acid sequence set forth in SEQ ID NO: 88;

(c) the heavy chain variable region comprising an amino acid sequence set forth in SEQ ID NO: 81, and the light chain variable region comprising an amino acid sequence set forth in SEQ ID NO: 82; or

(d) the heavy chain variable region comprising an amino acid sequence set forth in SEQ ID NO: 79, and the light chain variable region comprising an amino acid sequence set forth in SEQ ID NO: 80.

Embodiment 10. The pharmaceutical combination, method, or use according to any one of embodiments 1-3 and 8-9, wherein the anti-CD47 antibody comprises a heavy chain set forth in SEQ ID NO: 181 and a light chain set forth in SEQ ID NO: 183, or the anti-CD47 antibody comprises a heavy chain set forth in SEQ ID NO: 185 and a light chain set forth in SEQ ID NO: 187.

Embodiment 11. The pharmaceutical combination, use, or method according to any one of embodiments 1-10, wherein the anti-CD47 antibody is of an IgG1, IgG2, IgG3, or IgG4 subclass.

Embodiment 12. The use or method according to any one of embodiments 1-11, wherein the hematologic tumor is newly diagnosed, recurrent, or refractory.

Embodiment 13. The use or method according to any one of embodiments 1-12, wherein the hematologic tumor is leukemia, lymphoma, myelodysplastic syndrome, or myeloma; or the hematologic tumor is multiple myeloma, acute lymphoblastic leukemia, acute myeloid leukemia, chronic myeloid leukemia, rare types of leukemia, myelodysplastic syndrome, classical Hodgkin's lymphoma, nodular lymphocyte-predominant Hodgkin's lymphoma, diffuse large B-cell lymphoma, follicular lymphoma, mantle cell lymphoma, marginal zone lymphoma, peripheral T-cell lymphoma, extranodal NK/T-cell lymphoma, extranodal NK/T-cell lymphoma, nasal type, Burkitt lymphoma, chronic lymphocytic leukemia, primary central nerves system lymphoma, lymphoblastic lymphoma, AIDS-related B-cell lymphoma, chronic lymphocytic leukemia, small lymphocytic lymphoma, chronic lymphocytic leukemia/small lymphocytic lymphoma, cutaneous B-cell lymphoma, primary mediastinal large B-cell lymphoma, unclassifiable B-cell lymphoma, Waldenström's macroglobulinemia, or light chain amyloidosis.

Embodiment 14. The pharmaceutical combination, use, or method according to any one of embodiments 1-13, wherein the second therapeutic agent is selected from the group consisting of a hypomethylating agent, an anti-CD20 antibody, an immunomodulator, and a proteasome inhibitor.

Embodiment 15. The pharmaceutical combination, use, or method according to any one of embodiments 1-14, wherein the second therapeutic agent is a hypomethylating agent; preferably, the hypomethylating agent is selected from the group consisting of azacitadine, decitabine, guadecitabine, zebularine, and procaine.

Embodiment 16. The pharmaceutical combination, use, or method according to embodiment 15, wherein the hypomethylating agent is azacitadine.

Embodiment 17. The use or method according to embodiment 15 or 16, wherein the hematologic tumor is leukemia, myelodysplastic syndrome, lymphoma, or myeloma.

Embodiment 18. The use or method according to embodiment 17, wherein the hematologic tumor is acute myeloid leukemia (AML); preferably, the AML is AML with at least one genetic abnormality, AML with multilineage dysplasia, therapy-related AML, undifferentiated AML, AML with minimal maturation, AML with maturation, acute myelomonocytic leukemia, acute monocytic leukemia, acute erythroleukemia, acute megakaryoblastic leukemia, acute basophilic leukemia, acute panmyelosis with myelofibrosis, or myeloid sarcoma; preferably, the at least one genetic abnormality is selected from the group consisting of a translocation t(8;21)(q22;q22), an inversion inv(16)(p13;q22), a translocation t(16;16)(p13;q22), a translocation t(15;17)(q22;q12), a *PML/PARα* fusion, an *AML1/ETO* fusion, a *CBFB/MYH11* fusion, an *MLL* rearrangement, a *BCR/ABL1* fusion, a *C-KIT* mutation, an *FLT3* mutation, an *NPM1* mutation, a *CEBPA* mutation, a *TP53* mutation, an *RUNX1(AML1)* mutation, an *ASXLI* mutation, an *IDH1* mutation, an *IDH2* mutation, a *DNMT3a* mutation, a *TET2* mutation, an *SF3B1* mutation, a *U2AF1* mutation, an *SRSF2* mutation, a *ZRSR2* mutation, an *EZH2* mutation, a *BCOR* mutation, an *STAG2* mutation, a *CALR* mutation, an *MPL* mutation, a *CSF3R* mutation, a *CBL* mutation, an *SETBP1* mutation, an *SF3B1* mutation, a *U2AF1* mutation, a *WT1* mutation, a *CSF3R* mutation, a *JAK1* mutation, a *JAK2* mutation, a *JAK3* mutation, a *BRAF* mutation, a *TET2* mutation, a *KRAS* mutation, an *NRAS* mutation, and a *PTPN11* mutation.

Embodiment 19. The use or method according to embodiment 17, wherein the hematologic tumor is myelodysplastic syndrome (MDS); preferably, the MDS is MDS with single lineage dysplasia, MDS with multiple lineage dysplasia, MDS with ring sideroblasts, MDS with excess blasts, or MDS, unclassifiable.

Embodiment 20. The pharmaceutical combination, use, or method according to any one of embodiments 1-14, wherein the second therapeutic agent is an anti-CD20 antibody; preferably, the anti-CD20 antibody is obinutuzumab, ofatumumab, ocrelizumab, ibritumomab, tositumomab, rituximab, or ublituximab.

Embodiment 21. The use or method according to embodiment 20, wherein the anti-CD20 antibody is rituximab.

Embodiment 22. The use or method according to embodiment 20 or 21, wherein the hematologic tumor is leukemia, myelodysplastic syndrome, lymphoma, or myeloma.

Embodiment 23. The use or method according to embodiment 22, wherein the hematologic tumor is lymphoma; preferably, the lymphoma is classical Hodgkin's lymphoma, nodular lymphocyte-predominant Hodgkin's lymphoma, diffuse large B-cell lymphoma, follicular lymphoma, mantle cell lymphoma, marginal zone lymphoma, peripheral T-cell lymphoma, extranodal NK/T-cell lymphoma, extranodal NK/T-cell lymphoma, nasal type, Burkitt lymphoma, chronic lymphocytic leukemia, primary central nerves system lymphoma, lymphoblastic lymphoma, AIDS-related B-cell lymphoma, chronic lymphocytic leukemia, small lymphocytic lymphoma, chronic lymphocytic leukemia/small lymphocytic lymphoma, cutaneous B-cell lymphoma, primary mediastinal large B-cell lymphoma, or unclassifiable B-cell lymphoma.

Embodiment 24. The pharmaceutical combination, use, or method according to any one of embodiments 1-14, wherein the second therapeutic agent is a proteasome inhibitor or/and an immunomodulator.

Embodiment 25. The pharmaceutical combination, use, or method according to embodiment 24, wherein the proteasome inhibitor is bortezomib, carfilzomib, or ixazomib, preferably bortezomib.

Embodiment 26. The pharmaceutical combination, use, or method according to embodiment 24 or 25, wherein the immunomodulator is lenalidomide, thalidomide, or pomalidomide, preferably lenalidomide.

Embodiment 27. The use or method according to any one of embodiments 24-26, wherein the hematologic tumor is leukemia, myelodysplastic syndrome, lymphoma, or myeloma, and preferably, the myeloma is multiple myeloma.

Embodiment 28. The pharmaceutical combination, use, or method according to any one of embodiments 1-27, wherein the anti-CD47 antibody is at a therapeutic dose of 60 mg to 3000 mg.

Embodiment 29. The pharmaceutical combination, use, or method according to any one of embodiments 1-28, wherein the anti-CD47 antibody is administered in a therapeutic dose once every 3-21 days.

Embodiment 30. The pharmaceutical combination, use, or method according to any one of embodiments 1-29, wherein a sub-therapeutic dose of the anti-CD47 antibody is administered prior to administration of the therapeutic dose of the anti-CD47 antibody.

Embodiment 31. The pharmaceutical combination, use, or method according to any one of embodiments 1-30, wherein the anti-CD47 antibody is administered in a dose escalation manner to achieve the therapeutic dose.

Embodiment 32. The pharmaceutical combination, use, or method according to any one of embodiments 1-31, wherein the anti-CD47 antibody is administered intravenously.

Embodiment 33. The pharmaceutical combination, use, or method according to any one of embodiments 1-32, wherein the anti-CD47 antibody and the second therapeutic agent are each in the form of a pharmaceutical composition.

Embodiment 34. The pharmaceutical combination, use, or method according to any one of embodiments 1-33, wherein the anti-CD47 antibody and the second therapeutic agent are administered simultaneously, sequentially, or at an interval.

Embodiment 35. The pharmaceutical combination according to any one of embodiments 2-34, wherein the pharmaceutical combination is a non-fixed combination.

Embodiment 36. A kit comprising the pharmaceutical combination according to any one of embodiments 2-35.

Embodiment 37. Use of the anti-CD47 antibody for preparing a medicament for treating myeloma in a subject.

## DETAILED DESCRIPTION

### Example 1. Production of Anti-CD47 Monoclonal Antibodies

**Immunization of mice with CD47 protein and acquisition of antibody variable region sequences:**

To produce antibodies against CD47, Balb/c mice were immunized subcutaneously with a recombinant hCD47-His Tag protein (ACRObiosystems, Cat. No. CD7-H5227) (50 μg/Balb/c mice) or hCD47-mFc (ACRObiosystems, Cat. No. CD7-H52A5) emulsified in an equal volume of Freund's complete adjuvant (first immunization) or Freund's incomplete adjuvant (booster immunization) every 2 weeks for three times over a period of 6 weeks. In the fourth immunization, each mouse was injected intramuscularly with 20 μg of hCD47-mFc (ACRObiosystems, Cat. No. CD7-H52A5) protein (in aqueous adjuvant). Three days prior to cell fusion, mice were injected intravenously with an antigen without an adjuvant to boost the immunization. Spleen cells ($1 \times 10^8$) from the immunized mice were fused with SP2/0 myeloma cells ($1.5 \times 10^7$) using polyethylene glycol 1500 (Roche, Cat. No. 10783641001).

The cloned hybridoma cells were preserved and used for extraction of cDNA to obtain antibody variable region sequences. The heavy chain variable region (VH) sequences and light chain variable region (VL) sequences of five murine monoclonal antibodies (2B2, 2H8, 3F10, 16E5 and 14A9) were obtained from the corresponding hybridoma clones (2B2, 2H8, 3F10, 16E5 and 14A9) by amplification and sequencing. The information on the sequences is shown as follows:

| Name of antibody | VH(SEQ ID NO: ) | | VL(SEQ ID NO: ) | |
|---|---|---|---|---|
| | Amino acid sequence | Nucleotide sequence | Amino acid sequence | Nucleotide sequence |
| 2H8 | 33 | 189 | 34 | 190 |
| 16E5 | 35 | 191 | 36 | 192 |
| 2B2 | 37 | 193 | 38 | 194 |
| 3F10 | 39 | 195 | 40 | 196 |
| 14A9 | 41 | 197 | 42 | 198 |

**Construction and expression of chimeric antibodies:**

Through the chemical synthesis of genes of mouse antibody variable regions, the VL region of each mouse antibody was linked to the human κ chain constant region to construct a chimeric light chain, and the mouse VH region was linked to the IgG4 (with the EU number of S228P) constant region to construct a chimeric heavy chain. Expression plasmids for the chimeric light chain and the chimeric heavy chain were constructed by conventional genetic engineering means. CHO cells (a 200 mL system at $10^6$ cells/mL) were transfected with 100 μg of each of the expression plasmids for the chimeric heavy chain and the chimeric light chain, and then cultured for 6 days. Then the chimeric antibody in the supernatant was purified using a protein A column.

Five chimeric antibodies were obtained by construction and expression. The full-length sequences of the chimeric heavy chains and the chimeric light chains of these antibodies and the nucleic acids encoding the same were as follows: Xi2H8 (chimeric heavy chain and nucleic acid encoding the same: SEQ ID NOs: 89-90; chimeric light chain and nucleic acid encoding the same: SEQ ID NOs: 91-92), Xi2B2 (chimeric heavy chain and nucleic acid encoding the same: SEQ ID NOs: 97-98; chimeric light chain and nucleic acid encoding the same: SEQ ID NOs: 99-100), Xi3F10 (chimeric heavy chain and nucleic acid encoding the same: SEQ ID NOs: 101-102; chimeric light chain and nucleic acid encoding the same: SEQ ID NOs: 103-104), Xi16E5 (chimeric heavy chain and nucleic acid encoding the same: SEQ ID NOs: 93-94; chimeric light chain and nucleic acid encoding the same: SEQ ID NOs: 95-96), and Xi14A9 (chimeric heavy chain and nucleic acid encoding the same: SEQ ID NOs: 105-106; chimeric light chain and nucleic acid encoding the same: SEQ ID NOs: 107-108).

**Humanization design of antibodies:**

Human murine chimeric antibodies of 3F10, 14A9, and 16E5 were humanized by CDR grafting (see, e.g., U.S. Pat. No. 5,225,539).

The sequences of the humanized antibodies 3F10, 14A9, and 16E5 and the nucleic acids encoding the same were as follows: hz3F10-1.1 (heavy chain and nucleic acids encoding the same: SEQ ID NOs: 109-110; light chain and nucleic acids encoding the same: SEQ ID NOs: 111-112), hz3F10-2.1 (heavy chain and nucleic acids encoding the same: SEQ ID NOs: 113-114; light chain and nucleic acids encoding the same: SEQ ID NOs: 115-116), hz3F10-3.1 (heavy chain and nucleic acids encoding the same: SEQ ID NOs: 117-118; light chain and nucleic acids encoding the same: SEQ ID NOs: 119-120), hz3F10-4.1 (heavy chain and nucleic acids encoding the same: SEQ ID NOs: 121-122; light chain and nucleic acids encoding the same: SEQ ID NOs: 123-124), hz3F10-5.1 (heavy chain and nucleic acids encoding the same: SEQ ID NOs: 125-126; light chain and nucleic acids encoding the same: SEQ ID NOs: 127-128), hz3F10-6.1 (heavy chain and nucleic acids encoding the same: SEQ ID NOs: 129-130; light chain and nucleic acids encoding the same: SEQ ID NOs: 131-132), hz3F10-1.2 (heavy chain and nucleic acids encoding the same: SEQ ID NOs: 133-134; light chain and nucleic acids encoding the same: SEQ ID NOs: 135-136), hz3F10-2.2 (heavy chain and nucleic acids encoding the same: SEQ ID NOs: 137-138; light chain and nucleic acids encoding the same: SEQ ID NOs: 139-140), hz3F10-3.2 (heavy chain and nucleic acids encoding the same: SEQ ID NOs: 141-142; light chain and nucleic acids encoding the same: SEQ ID NOs: 143-144), hz3F10-4.2 (heavy chain and nucleic acids encoding the same: SEQ ID NOs: 145-146; light chain and nucleic acids encoding the same: SEQ ID NOs: 147-148), hz3F10-5.2 (heavy chain and nucleic acids encoding the same: SEQ ID NOs: 149-150; light chain and nucleic acids encoding the same: SEQ ID NOs: 151-152), hz3F10-6.2 (heavy chain and nucleic acids encoding the same: SEQ ID NOs: 153-154; light chain and nucleic acids encoding the same: SEQ ID NOs: 155-156), hz16E5-1.1 (heavy chain and nucleic acids encoding the same: SEQ ID NOs: 157-158; light chain and nucleic acids encoding the same: SEQ ID NOs: 159-160), hz16E5-1.3 (heavy chain and nucleic acids encoding the same: SEQ ID NOs: 161-162; light chain and nucleic acids encoding the same: SEQ ID NOs: 163-164), hz16E5-1.2 (heavy chain and nucleic acids encoding the same: SEQ ID NOs: 165-166; light chain and nucleic acids encoding the same: SEQ ID NOs: 167-168), hz16E5-3.2 (heavy chain and nucleic acids encoding the same: SEQ ID NOs: 169-170; light chain and nucleic acids encoding the same: SEQ ID NOs: 171-172), hz16E5-3.1 (heavy chain and nucleic acids encoding the same: SEQ ID NOs: 173-174; light chain and nucleic acids encoding the same: SEQ ID NOs: 175-176), hz16E5-3.3 (heavy chain and nucleic acids encoding the same: SEQ ID NOs: 177-178; light chain and nucleic acids encoding the same: SEQ ID NOs: 179-180), hz14A9-2.3 (heavy chain and nucleic acids encoding the same: SEQ ID NOs: 181-182; light chain and nucleic acids encoding the same: SEQ ID NOs: 183-184), and hz14A9-2.4 (heavy chain and nucleic acids encoding the same: SEQ ID NOs: 185-186; light chain and nucleic acids encoding the same: SEQ ID NOs: 187-188).

**Construction and expression of humanized antibodies 3F10, 14A9, and 16E5:**

DNAs encoding the full-length light chain and the full-length heavy chain of the humanized antibodies 3F10, 14A9, and 16E5 were synthesized and cloned into expression vectors (including, for example, pcDNA3.1 vector disclosed in CN107001463A, and pCHO1.0 vector disclosed in CN109422811A). CHO cells (a 200 mL system at $10^6$ cells/mL) were transfected with 100 μg of each of the expression plasmids for the heavy chain and light chain of the humanized antibodies, and then cultured in an ExpiCHO medium (Gibco, Cat. No. A29100-01) in an incubator at 37 °C with 5%

$CO_2$ for 6 days. After centrifugation, a supernatant was obtained, and the humanized antibodies in the supernatant were purified using a protein A column.

**Example 2. Biacore-Based Affinity Analysis of Anti-CD47 Antibodies**

The equilibrium dissociation constants $K_D$ (in unit M) were obtained by measuring the binding kinetics of the human CD47 protein (human CD47 protein, His Tag; ACRObiosystems, Cat. No. CD7-H5227) and cynomolgus monkey CD47 protein (cynomolgus/rhesus macaque CD47 protein, His Tag; ACRObiosystems, Cat. No. CD7-C52H1) to anti-CD47 antibodies (chimeric or humanized) using Biacore. The Biacore analysis was performed at 25 °C and data were recorded at a rate of 1 Hz.

Polyclonal rabbit anti-mouse IgG (GE, BR-1008-38) was diluted with 10 mM sodium acetate at pH 5.0 and immobilized onto the reference flowcell and experimental flowcell of a CM5 biosensor chip using an amine coupling kit (GE, BR10050), and about 15,000 RM was reached. At the beginning of each cycle, the diluted test antibody (1.5 μg/mL) was injected onto the experimental flowcell and maintained for 1 min to be captured. These data showed that the anti-CD47 antibodies (chimeric and humanized) bound to CD47 as measured by Biacore.

Table 1: Binding affinities of anti-CD47 chimeric antibodies for human CD47 and cynomolgus monkey CD47

| Name of antibody | Biacore: equilibrium dissociation constant $K_D$ (in unit M) | |
|---|---|---|
| | Human CD47 protein | Cynomolgus monkey CD47 protein |
| Xi2B2 | 7.48E-09 | 1.20E-09 |
| Xi2H8 | 3.50E-10 | 1.25E-09 |
| Xi3F10 | 7.67E-10 | 1.04E-09 |
| Xi16E5 | 9.06E-09 | 2.92E-09 |
| Xi14A9 | 2.35E-09 | 1.58E-09 |

Table 2: Binding affinities of anti-CD47 humanized antibodies for human CD47 and cynomolgus monkey CD47

| Name of antibody | Biacore: equilibrium dissociation constant $K_D$ (in unit M) | |
|---|---|---|
| | Human CD47 protein | Cynomolgus monkey CD47 protein |
| hz3F10-1.1 | 9.46E-10 | 1.31E-09 |
| hz3F10-2.1 | 8.36E-10 | 1.17E-09 |
| hz3F10-3.1 | 1.53E-09 | 1.92E-09 |
| hz3F10-4.1 | 1.42E-09 | 1.62E-09 |
| hz3F10-5.1 | 9.60E-10 | 1.30E-09 |
| hz3F10-6.1 | 5.92E-10 | 2.47E-09 |
| hz3F10-1.2 | 9.04E-10 | 1.33E-09 |
| hz3F10-2.2 | 7.79E-10 | 1.14E-09 |
| hz3F10-3.2 | 1.15E-09 | 1.47E-09 |
| hz3F10-4.2 | 1.34E-09 | 1.86E-09 |
| hz3F10-5.2 | 8.96E-10 | 1.40E-09 |
| hz3F10-6.2 | 7.65E-10 | 1.15E-09 |
| hz16E5-1.1 | 1.89E-08 | 4.77E-09 |
| hz16E5-3.1 | 6.28E-09 | 3.28E-09 |
| hz16E5-1.2 | 9.31E-09 | 2.26E-09 |
| hz16E5-3.2 | 1.71E-08 | 3.10E-09 |

(continued)

| Name of antibody | Biacore: equilibrium dissociation constant $K_D$ (in unit M) | |
| --- | --- | --- |
| | Human CD47 protein | Cynomolgus monkey CD47 protein |
| hz16E5-1.3 | 1.53E-08 | 2.73E-09 |
| hz16E5-3.3 | 7.13E-09 | 1.87E-09 |
| hz14A9-2.3 | 2.04E-10 | 3.07E-09 |
| hz14A9-2.4 | 8.55E-09 | 2.00E-09 |
| Hu5F9 | 8.33E-10 | 1.92E-09 |

**Example 3. Analysis of Anti-CD47 Antibodies Based on Promotion of Macrophage Phagocytosis**

The ability of the anti-CD47 antibodies to promote the phagocytosis of tumor cells by macrophages having SIRPα on the cell surface was determined based on flow cytometry.

Monocytes were isolated from human peripheral blood mononuclear cells (PBMC, Sailybio, Cat. No. 190056) using a monocyte isolation kit (STEMCELL, Cat. No. 19058), plated into a 24-well plate (Greiner bio-one, Cat. No. 662-160) at $5 \times 10^5$ cells per well, and induced under 100 ng/mL stimulating factor M-CSF (R&D Systems, Cat. No. 216-MC-010) for 7 days to form macrophages. $1 \times 10^6$ target cells HL60 (Shanghai Institute of Biochemistry and Cell Biology, Chinese Academy of Sciences, Cat. No. TCHu23) were suspended in 5 μmol/L CFSE (BD Biosciences, Cat. No. 565082) fluorescent dye (diluted with a PBS buffer to the desired concentration), incubated in a water bath at 37 °C in the dark for 15 min, and washed with a PBS buffer to prepare CFSE-labeled HL60 cells, which were then incubated with each of 10 μg/mL and 1 μg/mL anti-CD47 antibodies (see Table 3 for chimeric antibodies, and see Table 4 for humanized antibodies) in an incubator at 37 °C with 5% $CO_2$ for 1-2 h. The HL60 cells after the incubation were washed three times with a PBS buffer and added to the macrophages. The cells were incubated in an incubator at 37 °C with 5% $CO_2$ for 2-4 h. After the cells were washed three times with a PBS buffer, a mixture of 260 μL of PBS, 20 μL of anti-APC-CD14 (APC Mouse Anti-Human CD14, BD Biosciences, Cat. No. 555399), and 20 μL of PE-CD11b (PE Mouse Anti-Human CD11b/Mac-1, BD Biosciences, Cat. No. 555388) antibody was added to each well. The cells were incubated at 4 °C in the dark for 30 min, washed three times with a PBS buffer, and digested with trypsin (Gibco, Cat. No. 12604-013). The rate of phagocytosis (%) by the macrophages was determined using a flow cytometer (BD Biosciences, model: C6).

The effects of the anti-CD47 chimeric antibodies and anti-CD47 humanized antibodies on macrophage phagocytosis are shown in Tables 3 and 4. Among the antibodies, the chimeric antibodies Xi3F10, $X_1$16E5 and Xi14A9, and the anti-CD47 humanized antibodies had good promoting effects on phagocytosis, and the humanized antibodies hz14A9-2.3 and hz14A9-2.4 showed better performance.

Table 3: Analysis of anti-CD47 chimeric antibodies based on promotion of macrophage phagocytosis (rate of phagocytosis, %)

| Name of antibody | 10 μg/mL | 1 μg/mL |
| --- | --- | --- |
| Xi2B2 | 4.1 | 7.3 |
| Xi2H8 | 3.5 | 7.1 |
| Xi3F10 | 59.8 | 65.7 |
| Xi16E5 | 28.8 | 11.5 |
| Xi14A9 | 44.67 | 54.17 |

Table 4: Analysis of anti-CD47 humanized antibodies based on promotion of macrophage phagocytosis (rate of phagocytosis, %)

| Name of antibody | 10 μg/mL | 1 μg/mL | Name of antibody | 10 μg/mL | 1 μg/mL |
| --- | --- | --- | --- | --- | --- |
| hz3F10-1.1 | 43.10 | 41.25 | hz3F10-6.2 | 44.19 | 49.32 |
| hz3F10-2.1 | 55.23 | 47.24 | hz16E5-1.1 | 4.02 | 14.79 |
| hz3F10-3.1 | 58.72 | 50.60 | hz16E5-3.1 | 24.19 | 6.37 |

(continued)

| Name of antibody | 10 µg/mL | 1 µg/mL | Name of antibody | 10 µg/mL | 1 µg/mL |
|---|---|---|---|---|---|
| hz3F10-4.1 | 58.26 | 50.26 | hz16E5-1.2 | 17.83 | 5.1 |
| hz3F10-5.1 | 58.46 | 50.45 | hz16E5-3.2 | 2.03 | 3.02 |
| hz3F10-6.1 | 60.91 | 47.57 | hz16E5-1.3 | 4.33 | 3.03 |
| hz3F10-1.2 | 38.86 | 55.52 | hz16E5-3.3 | 4.7 | 11.62 |
| hz3F10-2.2 | 34.72 | 44.19 | hz14A9-2.3 | 74.93 | 71.48 |
| hz3F10-3.2 | 37.09 | 47.04 | hz14A9-2.4 | 81.66 | 81.65 |
| hz3F10-4.2 | 31.67 | 46.24 | Hu5F9 | 72.35 | 67.5 |
| hz3F10-5.2 | 29.6 | 47.06 | | | |

**Example 4. Thermostability Assay of Anti-CD47 Antibodies Based on Nano DSF**

The high-throughput protein stability analyzer Prometheus NT.48 is an instrument for acquiring various types of data such as protein molecular structure stability, aggregation stability, and colloid dispersion stability. The melting temperatures (Tm) and the aggregation temperatures (Tagg) of the anti-CD47 chimeric antibodies and the anti-CD47 humanized antibodies were determined using this instrument. The results are shown in Tables 5 and 6. The results showed that the anti-CD47 antibodies had good thermostability.

Table 5: Thermostability of anti-CD47 chimeric antibodies

| Name of antibody | Tm(°C) | Tagg(°C) |
|---|---|---|
| Xi2B2 | 62.99 | 61.34 |
| Xi2H8 | 69.77 | 68.57 |
| Xi3F10 | 70.51 | 70.59 |
| Xi16E5 | 69.89 | 73.39 |
| Xi14A9 | 66.73 | 75.75 |

Table 6: Thermostability of anti-CD47 humanized antibodies

| Name of antibody | Tm(°C) | Tagg(°C) | Name of antibody | Tm(°C) | Tagg(°C) |
|---|---|---|---|---|---|
| hz3F10-1.1 | 68.44 | 72.68 | hz14A9-2.4 | 68.3 | 68.3 |
| hz3F10-2.1 | 68.44 | 72.68 | hz3F10-4.2 | 68.48 | 61.13 |
| hz3F10-3.1 | 68.15 | 61.53 | hz3F10-5.2 | 68.69 | 62.67 |
| hz3F10-4.1 | 68.35 | 62.54 | hz3F10-6.2 | 68.64 | 61.91 |
| hz3F10-5.1 | 68.05 | 62.12 | hz16E5-1.1 | 68.21 | 61.87 |
| hz3F10-6.1 | 68.6 | 68.6 | hz16E5-3.1 | 69.20 | 61.84 |
| hz3F10-1.2 | 68.71 | 61.92 | hz16E5-1.2 | 69.54 | 61.90 |
| hz3F10-2.2 | 68.92 | 62.40 | hz16E5-3.2 | 69.10 | 61.44 |
| hz3F10-3.2 | 68.30 | 61.80 | hz16E5-1.3 | 68.90 | 73.77 |
| hz 14A9-2.3 | 68.10 | 73.42 | hz16E5-3.3 | 68.72 | 73.81 |

**Example 5. Pharmacodynamic Activity of Anti-CD47 Antibodies in Hematologic Tumor Mouse Model of Lymphoma Raji Cells**

A hematologic tumor mouse model was established using hematologic tumor Raji cells. The anti-CD47 antibodies were applied to the model for the evaluation of the pharmacodynamic activity.

A suspension of Raji cells in a PBS buffer was prepared at a concentration of $5 \times 10^6$ cells/mL, and was injected into female NOD/SCID mice via the tail vein at 0.1 mL/mouse. After 7 days, the mice were randomly divided into 4 groups of 10 each, which were group 1: IgG4 group; group 2: Hu5F9 group; group 3: hz3F10-6.1; and group 4: hz14A9-2.3. The administration was performed by intraperitoneal injection in doses of 10 mg/kg for 21 consecutive days. The survival time of the animals was observed.

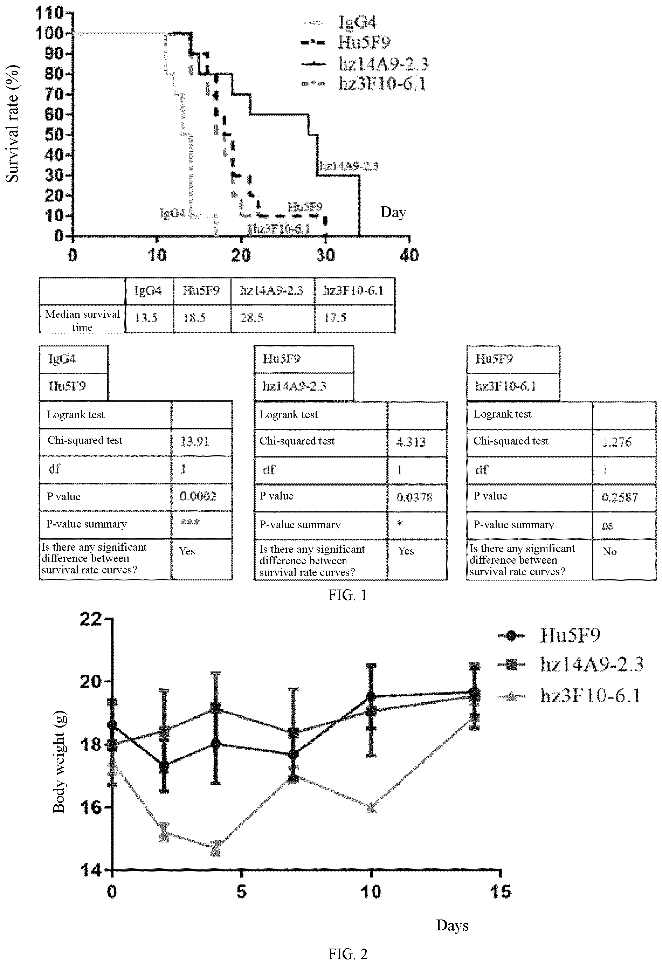

As shown in FIG. 1, the anti-CD47 antibodies hz14A9-2.3 and hz3F10-6.1 showed anti-tumor activity in the hematologic tumor model of Raji. The data for survival rates of the mice showed that: all the animals in the IgG4 group died on day 17, with a median survival time of 13.5 days; all the animals in the Hu5F9 group died on day 30, with a median survival time of 18.5 days; all the animals in the hz14A9-2.3 group died on day 34, with a median survival time of 28.5 days; all the animals in the hz3F10-6.1 group died on day 21, with a median survival time of 17.5 days. The data indicated that the antibody hz14A9-2.3 had better efficacy in prolonging the survival time of the animals than the other groups, and the antibodies hz3F10-6.1 and Hu5F9 had substantially equivalent effects.

**Example 6. Toxicity Assay of Anti-CD47 Antibodies in Single Administration**

Since CD47 is expressed on red cells and plays a role in clearing aged red cells, the toxic effects of the anti-CD47 antibodies in B-hCD47 mice were compared.

B-hCD47 mice were randomly divided into 3 groups: an Hu5F9 group of 3 mice, an hz14A9-2.3 group of 4 mice, and an hz3F10-6.1 group of 3 mice. The B-hCD47 mice were injected with an Hu5F9 antibody, an hz14A9-2.3 antibody, or an hz3F10-6.1 antibody via the tail vein in a single dose of 10 mg/kg, and changes in red blood cell count (RBC), hemoglobin (HGB) level, and body weight were determined 2, 4, 7, 10 and 14 days after administration.

Table 7: Rates of change in body weight of mice after administration (vs before administration)

| Days after administration | Hu5F9 10mpk | hz14A9-2.3 10mpk | hz3F10-6.1 10mpk |
| --- | --- | --- | --- |
| 0 | 0.0% | 0.0% | 0.0% |
| 2 | -7.0% | 2.3% | -13.0% |
| 4 | -3.2% | 6.3% | -15.8% |
| 7 | -5.1% | 2.0% | -2.5% |
| 10 | 4.8% | 5.9% | -8.4% |
| 14 | 5.6% | 8.6% | 8.2% |

Table 8: Rates of change in RBC of mice after administration (*vs* before administration

| Days after administration | Hu5F9 10mpk | hz14A9-2.3 10mpk | hz3F10-6.1 10mpk |
| --- | --- | --- | --- |
| 0 | 0.0% | 0.0% | 0.0% |
| 2 | -52.8% | -13.6% | -56.7% |
| 4 | -61.8% | -16.7% | -75.6% |
| 7 | -22.2% | -9.8% | -38.7% |
| 10 | -21.6% | -8.5% | -6.6% |
| 14 | -7.4% | -2.2% | -2.0% |

Table 9: Rates of change in HGB level of mice after administration (*vs* before administration

| Days after administration | Hu5F9 10mpk | hz14A9-2.3 10mpk | hz3F10-6.1 10mpk |
|---|---|---|---|
| 0 | 0.0% | 0.0% | 0.0% |
| 2 | -52.7% | -13.3% | -57.1% |
| 4 | -57.8% | -18.5% | -74.7% |
| 7 | -16.5% | -9.9% | -39.2% |
| 10 | -12.8% | -10.0% | -6.0% |
| 14 | -1.2% | -3.9% | -3.0% |

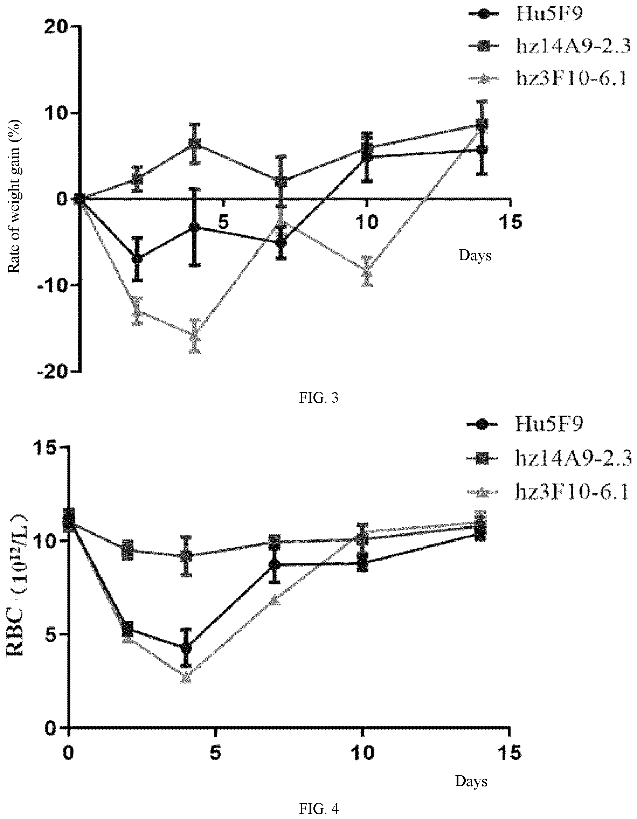

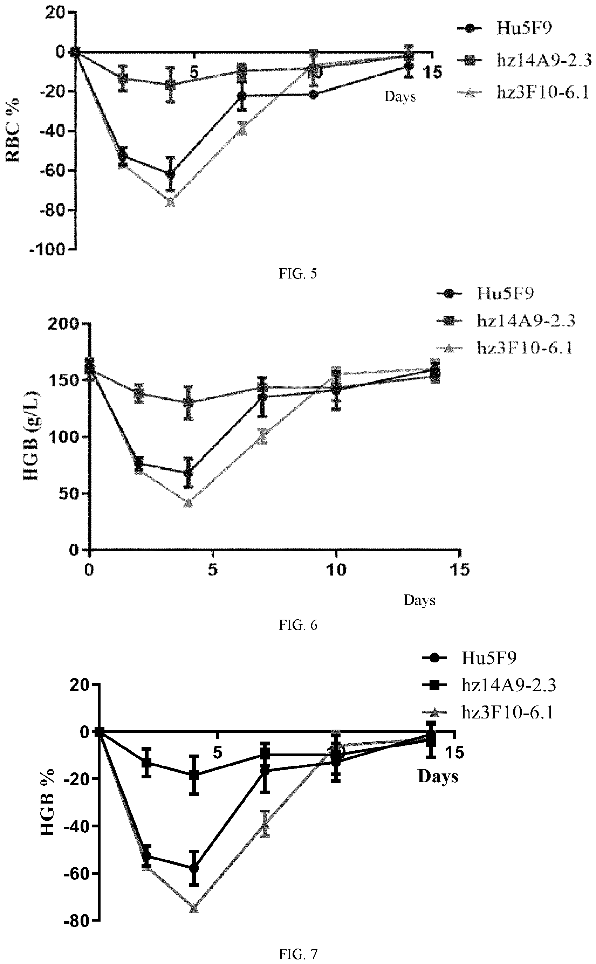

As shown in Table 7 and FIGs. 2 and 3, only mice in the hz14A9-2.3 group showed a continuous increase in body weight, while mice in the Hu5F9 group and the hz3F10-6.1 group showed a decrease in body weight first and then an increase after administration; the hz3F10-6.1 group showed a greater decrease than the Hu5F9 group. As shown in Tables 8-9 and FIGs. 4-7, the RBC and HGB levels in the animals of all the three groups decreased and reached a minimum 4 days after administration, and then began to increase. The RBC and HGB levels in the hz14A9-2.3 group were substantially normalized 7 days after administration, and those in the other two groups were substantially normalized at least 10 days after administration. Arranged in descending order according to the decreases in the RBC and HGB level 4 days after administration, the three groups are: hz3F10-6.1 > Hu5F9 > hz14A9-2.3. It could be seen that the antibody hz14A9-2.3 had no significant toxicity and was superior to the positive antibody Hu5F9.

**Example 7. Efficacy of Anti-CD47 Antibody and Azacitadine Alone or in Combination on Subcutaneous Mouse Xenograft Tumor of Human Acute Myeloblastic Leukemia MOLM-16**

The experimental animals were NOD-SCID mice (5-6 weeks old, female) purchased from Beijing Huafukang Biotechnology Co., Ltd., with the production license number of SCXK (Jing) 2019-0008 and the animal certification number of 110322211100910838. The housing environment was in a SPF grade. Human acute myelocytic leukemia MOLM-16 cells were purchased from DSMZ. MOLM-16 cells were cultured in an incubator at 37 °C with 5% $CO_2$ in air. Subculturing was performed twice a week, and when the cells were in a logarithmic growth phase, the cells were collected, counted, and inoculated. $1 \times 10^7$ MOLM-16 cells were inoculated to each mouse subcutaneously, and when tumors reached the volume of 100-150 $mm^3$, the mice were divided into groups and injected with drugs intravenously (IV) at the administration volume of 0.1 mL/10 g of body weight. The administration dosages and regimens are shown in Table 10.

The experimental index was to investigate the effects of the drugs on the tumor growth, and the specific index was T/C% or the tumor growth inhibition rate (TGI%, tumor growth inhibition).

Tumor diameters were measured twice a week with a vernier caliper and tumor volume (V) was calculated according to the following formula:

$$V = 1/2 \times a \times b^2,$$

where a and b represent length and width, respectively.

T/C (%) = (T - $T_0$)/(C - $C_0$) $\times$ 100, where T and C are the tumor volumes of animals at the end of the experiment in the test group and the negative control group, respectively; $T_0$ and $C_0$ are the tumor volumes of animals at the beginning of the experiment in the test group and the negative control group, respectively.

$$\text{Tumor growth inhibition \% (TGI\%)} = 100 - \text{T/C (\%)}.$$

When tumor regression occurred, tumor growth inhibition% (TGI%) = 100 - (T - $T_0$)/$T_0$ $\times$ 100.

If the volume of tumor shrinks compared with its initial volume, i.e., T < $T_0$ or C < $C_0$, it is defined as partial regression (PR) of tumor; if the tumor completely disappears, it is defined as complete regression (CR) of tumor. At the end of the experiment, the animals were sacrificed by $CO_2$ anesthesia and dissected to give the tumors. The tumors were photographed.

Table 10: Efficacy of anti-CD47 antibody and azacitadine used alone or in combination on subcutaneous mouse xenograft tumor of human acute myeloblastic leukemia MOLM-16

| Grouping | Time of administration | Administration Route | T/C (%) D12 | Tumor growth inhibition (%) D12 | Number of animals per group at the beginning of the experiment (mice) | Number of animals per group at the end of the experiment (mice) |
|---|---|---|---|---|---|---|
| hIgG4 0.5/1.5mg/kg | D0,3,6,10 | IV | - | - | 10 | 10 |
| hz14A9-2.3 0.5/1.5mg/kg | D0,3,6,10 | IV | 89 | 11 | 6 | 6 |
| Azacitadine 1 mg/kg | QD×5 | IV | 50 | 50 | 6 | 6 |
| Azacitadine 2.5 mg/kg | QD×5 | IV | 18 | 82 | 6 | 6 |
| hz14A9-2.3 0.5/1.5 mg/kg + Azacitadine 1 mg/kg | D0,3,6,10 + QD×5 | IV + IV | 42 | 58 | 6 | 6 |
| hz14A9-2.3 0.5/1.5 mg/kg + Azacitadine 2.5 mg/kg | D0,3,6,10 + QD×5 | IV + IV | 9 | 91 | 6 | 6 |

Note: The animals were randomly grouped, and the time for the first administration was D0; IV: intravenous injection; for hIgG4 and hz14A9-2.3, the first two administrations (D0 and D3) were performed at a dose of 0.5 mg/kg, and the last two administrations (D6 and D10) were performed at an adjusted dose of 1.5 mg/kg; QD: once daily.

The results are shown in Table 10. On D12 of the experiment, the tumor growth inhibition rate of hz14A9-2.3 (0.5/1.5 mg/kg, IV, D0, 3, 6, and 10) for the subcutaneous mouse xenograft tumor of human acute myeloblastic leukemia MOLM-16 was 11%; the tumor growth inhibition rates of azacitadine (1 mg/kg, IV, QD $\times$ 5) and azacitadine (2.5 mg/kg, IV, QD $\times$ 5) for the subcutaneous mouse xenograft tumor of MOLM-16 were 50% and 82%, respectively; the tumor growth inhibition rate of the combination of hz14A9-2.3 (0.5/1.5 mg/kg, IV, D0, 3, 6, and 10) and azacitadine (1 mg/kg, IV, QD $\times$ 5) for the subcutaneous mouse xenograft tumor of MOLM-16 was increased to 58%; the tumor growth inhibition rate of the combination of hz14A9-2.3 (0.5/1.5 mg/kg, IV, D0, 3, 6, and 10) and azacitadine (2.5 mg/kg, IV, QD $\times$ 5) for the subcutaneous mouse xenograft tumor of MOLM-16 was increased to 91% (D12).

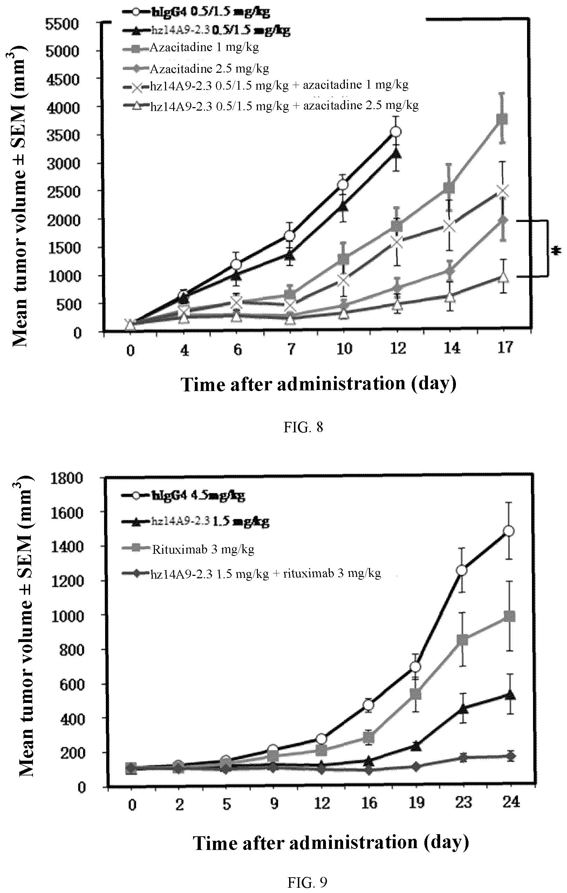

When the experiment was prolonged to D17, the mean tumor volumes of the azacitadine (1 or 2.5 mg/kg, IV, QD $\times$ 5) monotherapy groups were 3729.5 $\pm$ 436.4 mm$^3$ and 1920.7 $\pm$ 375.6 mm$^3$, respectively; the mean tumor volumes of the combinations of azacitadine and hz14A9-2.3 were 2447.0 $\pm$ 502.4 mm$^3$ and 922.4 $\pm$ 297.5 mm$^3$, respectively. Among these groups, the combination group of hz14A9-2.3 and 2.5 mg/kg azacitadine showed a significant increase in the tumor growth inhibition rate and was significantly stronger than the monotherapy group (P < 0.05, *vs* monotherapy) (FIG. 8). Furthermore, through the observation of the body weight of the mice, it was suggested that hz14A9-2.3 enhanced the efficacy of azacitadine on the subcutaneous mouse xenograft tumor of MOLM-16, and it did not significantly increase toxicity.

**Example 8. Efficacy of Anti-CD47 Antibody and Rituximab Alone or in Combination on Subcutaneous Mouse Xenograft Tumor of Human B-cell Lymphoma Daudi**

The experimental animals were NOD-SCID mice (5-6 weeks old, male) purchased from Shanghai Lingchang Biotechnology Co., Ltd., with the production license number of SCXK (Hu) 2018-0003 and the animal certification number of 20180003014870. The housing environment was in a SPF grade. Human B-cell lymphoma Daudi cells were purchased from Cell Bank of the Chinese Academy of Sciences. Daudi cells were cultured in an incubator at 37 °C with 5% $CO_2$ in air. Subculturing was performed twice a week, and when the cells were in a logarithmic growth phase, the cells were collected, counted, and inoculated. $1 \times 10^7$ Daudi cells were inoculated to each mouse subcutaneously, and when tumors reached the volume of about 100 mm$^3$, the mice were divided into groups and injected with drugs intravenously (IV) once every 3 days (Q3D) for a total of 2 times at the injection volume of 0.1 mL/10 g of body weight. The administration dosages and regimens are shown in Table 11. The experimental index was to investigate the effects of the drugs on the tumor growth, and the specific index was T/C% or the tumor growth inhibition rate (TGI%, tumor growth inhibition).

Tumor diameters were measured twice a week with a vernier caliper and tumor volume (V) was calculated according to the following formula:

$$V = 1/2 \times a \times b^2,$$

where a and b represent length and width, respectively.

T/C (%) = (T - $T_0$)/(C - $C_0$) × 100, where T and C are the tumor volumes of animals at the end of the experiment in the test group and the negative control group, respectively; $T_0$ and $C_0$ are the tumor volumes of animals at the beginning of the experiment in the test group and the negative control group, respectively.

$$\text{Tumor growth inhibition \% (TGI\%)} = 100 - \text{T/C (\%)}.$$

When tumor regression occurred, tumor growth inhibition% (TGI%) = 100 - (T - $T_0$)/$T_0$ × 100.

If the volume of tumor shrinks compared with its initial volume, i.e., T < $T_0$ or C < $C_0$, it is defined as partial regression (PR) of tumor; if the tumor completely disappears, it is defined as complete regression (CR) of tumor. At the end of the experiment (D24), the animals were sacrificed by $CO_2$ anesthesia and dissected to give the tumors. The tumors were photographed.

Table 11: Efficacy of anti-CD47 antibody and rituximab alone or in combination on subcutaneous xenograft tumor of human B-cell lymphoma Daudi

| Grouping | Time of administration | Administration Route | T/C (%) D24 | Tumor growth inhibition (%) D24 | Partial regression | Number of animals per group at the beginning of the experiment (mice) | Number of animals per group at the end of the experiment (mice) |
|---|---|---|---|---|---|---|---|
| hIgG4 4.5mg/kg | D0,3 | IV | - | - | 0 | 10 | 10 |
| hz14A9-2.3 1.5mg/kg | D0,3 | IV | 30 | 70 | 0 | 6 | 6 |
| Rituximab 3 mg/kg | D0,3 | IV | 64 | 36 | 0 | 6 | 6 |
| hz14A9-2.3 1.5 mg/kg + Rituximab 3 mg/kg | D0,3 + D0,3 | IV + IV | 4 | 96 | 2 | 6 | 6 |

Note: The animals were randomly grouped, and the time for the first administration was D0; IV: intravenous injection (IV).

The results are shown in Table 11 and FIG. 9. The anti-CD47 antibody hz14A9-2.3 (1.5 mg/kg, IV, D0 and 3) had a significant inhibitory effect on the growth of the subcutaneous mouse xenograft tumor of human B-cell lymphoma Daudi, with the tumor growth inhibition rate of 70%; the tumor growth inhibition rate of rituximab (3 mg/kg, IV, D0 and 3) for the subcutaneous mouse xenograft tumor of Daudi was 36%; the tumor growth inhibition rate of the combination of hz14A9-2.3 (1.5 mg/kg, IV, D0 and 3) and rituximab (3 mg/kg, IV, D0 and 3) were increased to 96%, with 2/6 of tumors partially regressed. The tumor-bearing mice were able to well tolerate the above drugs, without obvious symptoms such as weight loss. In comparison, the efficacy of the combination of hz14A9-2.3 and rituximab was significantly stronger than that of monotherapy (P < 0.01).

**Example 9. Phase I Clinical Trial of Anti-CD47 Antibody Alone or in Combination in Patients with Advanced Malignancies**

Primary objective: to evaluate the safety and tolerability of the anti-CD47 antibody alone (phase Ia) or in combination (phase Ib) in patients with advanced malignancies and determine the dose-limiting toxicity (DLT), maximum tolerated dose (MTD) (if any) or optimal biological dose (OBD), and recommended phase 2 dose (RP2D).

Secondary objectives: to evaluate the pharmacokinetic (PK) characteristics of the anti-CD47 antibody alone or in combination in patients with advanced malignancies; to evaluate the preliminary efficacy of the anti-CD47 antibody alone or in combination for advanced malignancies; to evaluate the immunogenicity of the anti-CD47 antibody; to evaluate the pharmacodynamic (PD) characteristics of the anti-CD47 antibody alone or in combination.

Principal enrollment criteria:

1. 18 years $\leq$ age $\leq$ 75 years (based on the date of signing an informed consent); male or female without limitation; ECOG score: 0-1; expected survival $\geq$ 3 months.
2. For lymphomas, the following criteria must be at least met:

Histologically well-diagnosed lymphoma, including, but not limited to, classical Hodgkin's lymphoma and non-Hodgkin's lymphoma.

CD20-positive by an immunophenotypic analysis (only for the phase Ib of combination dose escalation).

Prior $\geq$ second-line adequate treatment and failure to achieve remission on the last adequate treatment or disease progression/recurrence after achieving remission (including recurrence after receiving autologous hematopoietic stem cell transplantation), CD20-positive disease also refractory to at least one regimen of anti-CD20 monoclonal antibody alone or in combination with chemotherapy (except for chronic lymphocytic leukemia/small lymphocytic lymphoma); or no standard of care; or unwillingness to accept existing approved therapies.

3. For leukemias, the following criteria must be at least met:

Acute myeloid leukemia (AML) or intermediate- to high-risk (IPSS-R) myelodysplastic syndrome (MDS) pathologically confirmed according to the 2016 World Health Organization (WHO) diagnostic criteria.

Recurrent/refractory disease, or intolerance to hypomethylating drugs or other drug treatments; or no other suitable treatment; or unwillingness to accept existing approved treatments.

4. For myelomas, the following criteria must be at least met:

Histologically confirmed multiple myeloma (MM).

Recurrent/refractory disease that has failed prior induction or consolidation or maintenance treatment, or intolerance to existing treatments; or no other suitable treatment; or unwillingness to accept existing approved treatments.

Experimental design and administration regimen:

This study was designed as an open-label, dose-escalation trial, including two phases of Ia of single dose escalation and Ib of combination dose escalation.

In the phase Ia of single dose escalation, eligible patients were sequentially assigned to 5 anti-CD47 antibody dose groups (60 mg, 180 mg, 600 mg, 1200 mg, and 1800 mg) in the order of enrollment. Each enrolled patient received the treatment with a target dose of the anti-CD47 antibody, tentatively by intravenous infusion once a week in one treatment cycle of every 4 weeks (28 days), for a maximum duration of treatment of no more than 2 years.

In the phase Ib of combination dose escalation, the MTD or OBD or RP2D dose or the administration regimen determined in the phase Ia of single dose escalation was selected, and the combination dose escalation was performed according to different indication cohorts. The patients with recurrent/refractory acute myeloid leukemia (AML) or myelodysplastic syndrome (MDS) were included in the cohort of the combination treatment of the anti-CD47 antibody and azacitadine, and the drug azacitadine for injection was a lyophilized powder injection for subcutaneous injection with the specification of 100 mg/vial, which was produced and supplied by Chia Tai Tianqing Pharmaceutical Group Co., Ltd. The patients with recurrent/refractory CD20-positive lymphoma were included in the cohort of the combination treatment of the anti-CD47 antibody and rituximab, and the drug rituximab for injection was a sterile injection for intravenous administration with the specification of 100 mg/10 mL, which was supplied by Nanjing Shunxin Pharmaceutical Co., Ltd. of Chia Tai Tianqing Pharmaceutical Group. The patients with recurrent/refractory multiple myeloma were included in the cohort of the combination of the anti-CD47 antibody and a proteasome inhibitor (bortezomib and the like) and/or an immunomodulator (lenalidomide and the like), and the drug bortezomib for injection was a lyophilized powder injection for intravenous administration (with the specification of 1 mg/vial and 3.5 mg/vial), which was produced and supplied by Chia Tai Tianqing Pharmaceutical Group Co., Ltd., and the drug lenalidomide capsule was an oral capsule with the specification of 25 mg, which was supplied by Nanjing Shunxin Pharmaceutical Co., Ltd. of Chia Tai Tianqing Pharmaceutical Group.

The inclusion/exclusion criteria, administration regimen, and study procedure of the phase Ib of combination dose escalation in the protocol would be revised in time according to the results and experience of the phase Ia of single dose escalation phase.

The evaluation of efficacy and safety was performed according to the standard.

The anti-CD47 antibody used in this study was an hz14A9-2.3 injection, which was a sterile injection for intravenous administration, and was produced and supplied by Nanjing Shunxin Pharmaceutical Co., Ltd. of Chia Tai Tianqing Pharmaceutical Group. This study also relates to the evaluation of the combination of the anti-CD47 antibody and other therapeutic agents, which is not described here.

**Claims**

1. Use of an anti-CD47 antibody and a second therapeutic agent for preparing a medicament for treating a hematologic tumor in a subject.

2. A pharmaceutical combination comprising an anti-CD47 antibody and a second therapeutic agent.

3. The pharmaceutical combination or use according to claim 1 or 2, wherein the anti-CD47 antibody comprises:

    (i) a heavy chain variable region comprising:

    HCDR1 comprising an amino acid sequence set forth in SEQ ID NO: 5, HCDR2 comprising an amino acid sequence set forth in SEQ ID NO: 10, and HCDR3 comprising an amino acid sequence set forth in SEQ ID NO: 15; and
    a light chain variable region comprising:
    LCDR1 comprising an amino acid sequence set forth in SEQ ID NO: 20, LCDR2 comprising an amino acid sequence set forth in SEQ ID NO: 25, and LCDR3 comprising an amino acid sequence set forth in SEQ ID NO: 30;

    (ii) a heavy chain variable region comprising:

    HCDR1 comprising an amino acid sequence set forth in SEQ ID NO: 1, HCDR2 comprising an amino acid sequence set forth in SEQ ID NO: 6, and HCDR3 comprising an amino acid sequence set forth in SEQ ID NO: 11; and
    a light chain variable region comprising: LCDR1 comprising an amino acid sequence set forth in SEQ ID NO: 16, LCDR2 comprising an amino acid sequence set forth in SEQ ID NO: 21, and LCDR3 comprising an amino acid sequence set forth in SEQ ID NO: 26;

    (iii) a heavy chain variable region comprising:

    HCDR1 comprising an amino acid sequence set forth in SEQ ID NO: 2, HCDR2 comprising an amino acid sequence set forth in SEQ ID NO: 7, and HCDR3 comprising an amino acid sequence set forth in SEQ ID NO: 12; and
    a light chain variable region comprising: LCDR1 comprising an amino acid sequence set forth in SEQ ID NO: 17, LCDR2 comprising an amino acid sequence set forth in SEQ ID NO: 22, and LCDR3 comprising an amino acid sequence set forth in SEQ ID NO: 27;

    (iv) a heavy chain variable region comprising:

    HCDR1 comprising an amino acid sequence set forth in SEQ ID NO: 3, HCDR2 comprising an amino acid sequence set forth in SEQ ID NO: 8, and HCDR3 comprising an amino acid sequence set forth in SEQ ID NO: 13; and
    a light chain variable region comprising: LCDR1 comprising an amino acid sequence set forth in SEQ ID NO: 18, LCDR2 comprising an amino acid sequence set forth in SEQ ID NO: 23, and LCDR3 comprising an amino acid sequence set forth in SEQ ID NO: 28; or

(v) a heavy chain variable region comprising:

HCDR1 comprising an amino acid sequence set forth in SEQ ID NO: 4, HCDR2 comprising an amino acid sequence set forth in SEQ ID NO: 9, and HCDR3 comprising an amino acid sequence set forth in SEQ ID NO: 14; and
a light chain variable region comprising:
LCDR1 comprising an amino acid sequence set forth in SEQ ID NO: 19, LCDR2 comprising an amino acid sequence set forth in SEQ ID NO: 24, and LCDR3 comprising an amino acid sequence set forth in SEQ ID NO: 32.

4. The pharmaceutical combination or use according to any one of claims 1-3, wherein the anti-CD47 antibody comprises a heavy chain variable region and a light chain variable region selected from any one of the following:

(a) the heavy chain variable region comprising an amino acid sequence having at least 80% identity to a sequence set forth in SEQ ID NO: 87, and the light chain variable region comprising an amino acid sequence having at least 80% identity to a sequence set forth in SEQ ID NO: 88;
(b) the heavy chain variable region comprising an amino acid sequence having at least 80% identity to a sequence set forth in SEQ ID NO: 33, and the light chain variable region comprising an amino acid sequence having at least 80% identity to a sequence set forth in SEQ ID NO: 34;
(c) the heavy chain variable region comprising an amino acid sequence having at least 80% identity to a sequence set forth in SEQ ID NO: 35, and the light chain variable region comprising an amino acid sequence having at least 80% identity to a sequence set forth in SEQ ID NO: 36;
(d) the heavy chain variable region comprising an amino acid sequence having at least 80% identity to a sequence set forth in SEQ ID NO: 37, and the light chain variable region comprising an amino acid sequence having at least 80% identity to a sequence set forth in SEQ ID NO: 38;
(e) the heavy chain variable region comprising an amino acid sequence having at least 80% identity to a sequence set forth in SEQ ID NO: 39, and the light chain variable region comprising an amino acid sequence having at least 80% identity to a sequence set forth in SEQ ID NO: 40;
(f) the heavy chain variable region comprising an amino acid sequence having at least 80% identity to a sequence set forth in SEQ ID NO: 41, and the light chain variable region comprising an amino acid sequence having at least 80% identity to a sequence set forth in SEQ ID NO: 42;
(g) the heavy chain variable region comprising an amino acid sequence having at least 80% identity to a sequence set forth in SEQ ID NO: 83, and the light chain variable region comprising an amino acid sequence having at least 80% identity to a sequence set forth in SEQ ID NO: 84; and
(h) the heavy chain variable region comprising an amino acid sequence having at least 80% identity to a sequence set forth in SEQ ID NO: 85, and the light chain variable region comprising an amino acid sequence having at least 80% identity to a sequence set forth in SEQ ID NO: 86.

5. The pharmaceutical combination or use according to any one of claims 1-4, wherein the anti-CD47 antibody is selected from any one of the following:

the anti-CD47 antibody comprising a heavy chain set forth in SEQ ID NO: 181 and a light chain set forth in SEQ ID NO: 183;
the anti-CD47 antibody comprising a heavy chain set forth in SEQ ID NO: 89 and a light chain set forth in SEQ ID NO: 91;
the anti-CD47 antibody comprising a heavy chain set forth in SEQ ID NO: 93 and a light chain set forth in SEQ ID NO: 95;
the anti-CD47 antibody comprising a heavy chain set forth in SEQ ID NO: 97 and a light chain set forth in SEQ ID NO: 99;
the anti-CD47 antibody comprising a heavy chain set forth in SEQ ID NO: 101 and a light chain set forth in SEQ ID NO: 103;
the anti-CD47 antibody comprising a heavy chain set forth in SEQ ID NO: 105 and a light chain set forth in SEQ ID NO: 107;
the anti-CD47 antibody comprising a heavy chain set forth in SEQ ID NO: 109 and a light chain set forth in SEQ ID NO: 111;

the anti-CD47 antibody comprising a heavy chain set forth in SEQ ID NO: 113 and a light chain set forth in SEQ ID NO: 115;

the anti-CD47 antibody comprising a heavy chain set forth in SEQ ID NO: 117 and a light chain set forth in SEQ ID NO: 119;

the anti-CD47 antibody comprising a heavy chain set forth in SEQ ID NO: 121 and a light chain set forth in SEQ ID NO: 123;

the anti-CD47 antibody comprising a heavy chain set forth in SEQ ID NO: 125 and a light chain set forth in SEQ ID NO: 127;

the anti-CD47 antibody comprising a heavy chain set forth in SEQ ID NO: 129 and a light chain set forth in SEQ ID NO: 131;

the anti-CD47 antibody comprising a heavy chain set forth in SEQ ID NO: 133 and a light chain set forth in SEQ ID NO: 135;

the anti-CD47 antibody comprising a heavy chain set forth in SEQ ID NO: 137 and a light chain set forth in SEQ ID NO: 139;

the anti-CD47 antibody comprising a heavy chain set forth in SEQ ID NO: 141 and a light chain set forth in SEQ ID NO: 143;

the anti-CD47 antibody comprising a heavy chain set forth in SEQ ID NO: 145 and a light chain set forth in SEQ ID NO: 147;

the anti-CD47 antibody comprising a heavy chain set forth in SEQ ID NO: 149 and a light chain set forth in SEQ ID NO: 151;

the anti-CD47 antibody comprising a heavy chain set forth in SEQ ID NO: 153 and a light chain set forth in SEQ ID NO: 155;

the anti-CD47 antibody comprising a heavy chain set forth in SEQ ID NO: 157 and a light chain set forth in SEQ ID NO: 159;

the anti-CD47 antibody comprising a heavy chain set forth in SEQ ID NO: 161 and a light chain set forth in SEQ ID NO: 163;

the anti-CD47 antibody comprising a heavy chain set forth in SEQ ID NO: 165 and a light chain set forth in SEQ ID NO: 167;

the anti-CD47 antibody comprising a heavy chain set forth in SEQ ID NO: 169 and a light chain set forth in SEQ ID NO: 171;

the anti-CD47 antibody comprising a heavy chain set forth in SEQ ID NO: 173 and a light chain set forth in SEQ ID NO: 175;

the anti-CD47 antibody comprising a heavy chain set forth in SEQ ID NO: 177 and a light chain set forth in SEQ ID NO: 179;

or

the anti-CD47 antibody comprising a heavy chain set forth in SEQ ID NO: 185 and a light chain set forth in SEQ ID NO: 187.

6. The use according to any one of claims 1-5, wherein the hematologic tumor is leukemia, lymphoma, myelodysplastic syndrome, or myeloma; or the hematologic tumor is multiple myeloma, acute lymphoblastic leukemia, acute myeloid leukemia, chronic myeloid leukemia, rare types of leukemia, myelodysplastic syndrome, classical Hodgkin's lymphoma, nodular lymphocyte-predominant Hodgkin's lymphoma, diffuse large B-cell lymphoma, follicular lymphoma, mantle cell lymphoma, marginal zone lymphoma, peripheral T-cell lymphoma, extranodal NK/T-cell lymphoma, extranodal NK/T-cell lymphoma, nasal type, Burkitt lymphoma, chronic lymphocytic leukemia, primary central nerves system lymphoma, lymphoblastic lymphoma, AIDS-related B-cell lymphoma, chronic lymphocytic leukemia, small lymphocytic lymphoma, chronic lymphocytic leukemia/small lymphocytic lymphoma, cutaneous B-cell lymphoma, primary mediastinal large B-cell lymphoma, unclassifiable B-cell lymphoma, Waldenström's macroglobulinemia, or light chain amyloidosis.

7. The pharmaceutical combination or use according to any one of claims 1-6, wherein the second therapeutic agent is selected from the group consisting of a hypomethylating agent, an anti-CD20 antibody, an immunomodulator, and a proteasome inhibitor.

8. The pharmaceutical combination or use according to any one of claims 1-7, wherein the second therapeutic agent is a hypomethylating agent; preferably, the hypomethylating agent is selected from the group consisting of azacitadine, decitabine, guadecitabine, zebularine, and procaine, preferably azacitadine.

9. The use according to claim 8, wherein the hematologic tumor is acute myeloid leukemia (AML); preferably, the AML is AML with at least one genetic abnormality, AML with multilineage dysplasia, therapy-related AML, undifferentiated AML, AML with minimal maturation, AML with maturation, acute myelomonocytic leukemia, acute monocytic leukemia, acute erythroleukemia, acute megakaryoblastic leukemia, acute basophilic leukemia, acute panmyelosis with myelofibrosis, or myeloid sarcoma; preferably, the at least one genetic abnormality is selected from the group consisting of a translocation t(8;21)(q22;q22), an inversion inv(16)(p13;q22), a translocation t(16;16)(p13;q22), a translocation t(15;17)(q22;q12), a *PML*/*RAR*α fusion, an *AML1*/*ETO* fusion, a *CBFb*/*MYH11* fusion, an *MLL* rearrangement, a *BCR*/*ABL1* fusion, a *C-KIT* mutation, an *FLT3* mutation, an *NPM1* mutation, a *CEBPA* mutation, a *TP53* mutation, an *RUNX1(AML1)* mutation, an *ASXLI* mutation, an *IDH1* mutation, an *IDH2* mutation, a *DNMT3a* mutation, a *TET2* mutation, an *SF3B1* mutation, a *U2AF1* mutation, an *SRSF2* mutation, a *ZRSR2* mutation, an *EZH2* mutation, a *BCOR* mutation, an *STAG2* mutation, a *CALR* mutation, an *MPL* mutation, a *CSF3R* mutation, a *CBL* mutation, an *SETBP1* mutation, an *SF3B1* mutation, a *U2AF1* mutation, a *WT1* mutation, a *CSF3R* mutation, a *JAK1* mutation, a *JAK2* mutation, a *JAK3* mutation, a *BRAF* mutation, a *TET2* mutation, a *KRAS* mutation, an *NRAS* mutation, and a *PTPN11* mutation.

10. The use according to claim 8, wherein the hematologic tumor is myelodysplastic syndrome (MDS); preferably, the MDS is MDS with single lineage dysplasia, MDS with multiple lineage dysplasia, MDS with ring sideroblasts, MDS with excess blasts, or MDS, unclassifiable.

11. The pharmaceutical combination or use according to any one of claims 1-7, wherein the second therapeutic agent is an anti-CD20 antibody; preferably, the anti-CD20 antibody is obinutuzumab, ofatumumab, ocrelizumab, ibritumomab, tositumomab, rituximab, or ublituximab;
more preferably, the anti-CD20 antibody is rituximab.

12. The use according to claim 11, wherein the hematologic tumor is lymphoma; preferably, the lymphoma is classical Hodgkin's lymphoma, nodular lymphocyte-predominant Hodgkin's lymphoma, diffuse large B-cell lymphoma, follicular lymphoma, mantle cell lymphoma, marginal zone lymphoma, peripheral T-cell lymphoma, extranodal NK/T-cell lymphoma, extranodal NK/T-cell lymphoma, nasal type, Burkitt lymphoma, chronic lymphocytic leukemia, primary central nerves system lymphoma, lymphoblastic lymphoma, AIDS-related B-cell lymphoma, chronic lymphocytic leukemia, small lymphocytic lymphoma, chronic lymphocytic leukemia/small lymphocytic lymphoma, cutaneous B-cell lymphoma, primary mediastinal large B-cell lymphoma, or unclassifiable B-cell lymphoma.

13. The pharmaceutical combination or use according to any one of claims 1-7, wherein the second therapeutic agent is a proteasome inhibitor or/and an immunomodulator;

preferably, the proteasome inhibitor is bortezomib, carfilzomib, or ixazomib, and more preferably bortezomib;
preferably, the immunomodulator is lenalidomide, thalidomide, or pomalidomide, and more preferably lenalidomide.

14. The use according to claim 13, wherein the hematologic tumor is multiple myeloma.

15. A kit comprising the pharmaceutical combination according to any one of claims 2-14.

| | IgG4 | Hu5F9 | hz14A9-2.3 | hz3F10-6.1 |
|---|---|---|---|---|
| Median survival time | 13.5 | 18.5 | 28.5 | 17.5 |

| IgG4 | |
|---|---|
| Hu5F9 | |
| Logrank test | |
| Chi-squared test | 13.91 |
| df | 1 |
| P value | 0.0002 |
| P-value summary | *** |
| Is there any significant difference between survival rate curves? | Yes |

| Hu5F9 | |
|---|---|
| hz14A9-2.3 | |
| Logrank test | |
| Chi-squared test | 4.313 |
| df | 1 |
| P value | 0.0378 |
| P-value summary | * |
| Is there any significant difference between survival rate curves? | Yes |

| Hu5F9 | |
|---|---|
| hz3F10-6.1 | |
| Logrank test | |
| Chi-squared test | 1.276 |
| df | 1 |
| P value | 0.2587 |
| P-value summary | ns |
| Is there any significant difference between survival rate curves? | No |

FIG. 1

FIG. 2

FIG. 3

FIG. 4

FIG. 5

FIG. 6

FIG. 7

FIG. 8

FIG. 9

## INTERNATIONAL SEARCH REPORT

| International application No. |
| --- |
| **PCT/CN2022/122513** |

### A. CLASSIFICATION OF SUBJECT MATTER

C07K 16/28(2006.01)i; A61K 39/395(2006.01)i; A61P 35/00(2006.01)i

According to International Patent Classification (IPC) or to both national classification and IPC

### B. FIELDS SEARCHED

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, DPWI, VEN, CNKI, Web of Science, 百度学术, BAIDU SCHOLAR, incoPat, 中国生物序列检索系统, Chinese Biological Sequence Retrieval System, NCBI, EBI, STNext: 申请人, 发明人, CD47, MER6, OA3, IAP, 血液肿瘤, 白血病, 淋巴瘤, 骨髓瘤, hemooncology, leukemia, lymphoma, myeloma, 阿扎胞苷, 地西他滨, 瓜地西他滨, 泽布拉林, 普鲁卡因, azacitidine, decitabine, zebularine, procaine, ethocaine, CD20抗体, 奥滨尤妥珠单抗, 奥法木单抗, 奥瑞珠单抗, 替伊莫单抗, 托西莫单抗, 利妥昔单抗, 乌妥昔单抗, CD20 antibody, Obinutuzumab, Ofatumumab, Ocrelizumab, Ibritumomab, Tositumomab, Rituximab, Ublituximab, 硼替佐米, 卡非佐米, 伊沙佐米, bortezomib, carfilzomib, ixazomib, 来那度胺, 沙利度胺, 泊马度胺, lenalidomide, thalidomide, pomalidomide, 序列1-198

### C. DOCUMENTS CONSIDERED TO BE RELEVANT

| Category* | Citation of document, with indication, where appropriate, of the relevant passages | Relevant to claim No. |
| --- | --- | --- |
| PX | WO 2021197401 A1 (CHIA TAI TIANQING PHARMACEUTICAL GROUP CO., LTD.) 07 October 2021 (2021-10-07)<br>claims 1-26 | 1, 2, 6-15 |
| X | WO 2021080920 A2 (ARCH ONCOLOGY, INC. et al.) 29 April 2021 (2021-04-29)<br>claims 1-44, and description, paragraphs 69-70 and 298-299 | 1, 2, 6-15 |
| X | WO 2021087064 A1 (FORTY SEVEN, INC.) 06 May 2021 (2021-05-06)<br>claims 1-133 | 1, 2, 6-15 |
| X | WO 2021076908 A1 (FORTY SEVEN, INC.) 22 April 2021 (2021-04-22)<br>claims 1-107 | 1, 2, 6-15 |
| X | WO 2020242895 A1 (FORTY SEVEN, INC.) 03 December 2020 (2020-12-03)<br>claims 1-47 | 1, 2, 6-15 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 November 2022** | **30 November 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/122513** |

**C. DOCUMENTS CONSIDERED TO BE RELEVANT**

| Category* | Citation of document, with indication, where appropriate, of the relevant passages | Relevant to claim No. |
| --- | --- | --- |
| X | DONIO, M. J. et al. "Pre-Clinical Combination of AO-176, a Highly Differentiated Clinical Stage CD47 Antibody, with Either Azacitidine or Venetoclax Significantly Enhances DAMP Induction and Phagocytosis of Acute Myeloid Leukemia." *Blood.*, Vol. 136, No. Supplement 1, 05 November 2020 (2020-11-05), article number: 9 | 1, 2, 6-15 |
| X | MAUTE, R. L. et al. "Translational Study of Cell Surface Proteins in Non-Hodgkin Lymphoma Patients Treated with the First-in-Class Anti-CD47 Antibody Magrolimab (5F9) in Combination with Rituximab." *Blood.*, Vol. 134, No. Supplement 1, 13 November 2019 (2019-11-13), article number: 9 | 1, 2, 6-15 |
| A | CN 111417653 A (HUMMINGBIRD BIOSCIENCE HOLDINGS PTE LTD.) 14 July 2020 (2020-07-14) entire document | 1-15 |

Form PCT/ISA/210 (second sheet) (January 2015)

**INTERNATIONAL SEARCH REPORT**

International application No.

**PCT/CN2022/122513**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   [1] The actually submitted sequence table is an XML file of the standard ST.26.

Form PCT/ISA/210 (continuation of first sheet) (January 2015)

**INTERNATIONAL SEARCH REPORT**
**Information on patent family members**

International application No.

**PCT/CN2022/122513**

| Patent document cited in search report | | | Publication date (day/month/year) | Patent family member(s) | | | Publication date (day/month/year) |
|---|---|---|---|---|---|---|---|
| WO | 2021197401 | A1 | 07 October 2021 | TW | 202144407 | A | 01 December 2021 |
| WO | 2021080920 | A2 | 29 April 2021 | EP | 4048406 | A2 | 31 August 2022 |
| | | | | US | 2022313819 | A1 | 06 October 2022 |
| | | | | CN | 114901364 | A | 12 August 2022 |
| | | | | AU | 2020372327 | A1 | 02 June 2022 |
| | | | | CA | 3158622 | A1 | 29 April 2021 |
| WO | 2021087064 | A1 | 06 May 2021 | TW | 202123971 | A | 01 July 2021 |
| | | | | EP | 4052040 | A1 | 07 September 2022 |
| | | | | CA | 3153636 | A1 | 06 May 2021 |
| | | | | IL | 292197 | A | 01 June 2022 |
| | | | | KR | 20220091576 | A | 30 June 2022 |
| | | | | US | 2021147568 | A1 | 20 May 2021 |
| | | | | AU | 2020374947 | A1 | 31 March 2022 |
| | | | | CN | 114599392 | A | 07 June 2022 |
| WO | 2021076908 | A1 | 22 April 2021 | CA | 3153501 | A1 | 22 April 2021 |
| | | | | AU | 2020365113 | A1 | 07 April 2022 |
| | | | | KR | 20220085796 | A | 22 June 2022 |
| | | | | TW | 202124450 | A | 01 July 2021 |
| | | | | CN | 114555123 | A | 27 May 2022 |
| | | | | US | 2021115140 | A1 | 22 April 2021 |
| | | | | IL | 292162 | A | 01 June 2022 |
| | | | | EP | 4045083 | A1 | 24 August 2022 |
| WO | 2020242895 | A1 | 03 December 2020 | EP | 3976658 | A1 | 06 April 2022 |
| | | | | KR | 20220012328 | A | 03 February 2022 |
| | | | | CA | 3140639 | A1 | 03 December 2020 |
| | | | | AU | 2020283811 | A1 | 16 December 2021 |
| | | | | TW | 202110886 | A | 16 March 2021 |
| | | | | CN | 113906049 | A | 07 January 2022 |
| | | | | JP | 2022533253 | A | 21 July 2022 |
| | | | | US | 2020369767 | A1 | 26 November 2020 |
| CN | 111417653 | A | 14 July 2020 | US | 2022298254 | A1 | 22 September 2022 |
| | | | | JP | 2021500926 | A | 14 January 2021 |
| | | | | AU | 2018358004 | A1 | 28 May 2020 |
| | | | | CA | 3080860 | A1 | 09 May 2019 |
| | | | | KR | 20200079511 | A | 03 July 2020 |
| | | | | WO | 2019086573 | A1 | 09 May 2019 |
| | | | | EP | 3704157 | A1 | 09 September 2020 |
| | | | | SG | 11202003943Y | A | 28 May 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5225539 A
- CN 107001463 A
- CN 109422811 A

**Non-patent literature cited in the description**

- **ELVIN A. KABAT et al.** Sequences of Proteins of Immunological Interest. Public Health Service, 1991
- **CYRUS CHOTHIA et al.** Canonical Structures for the Hypervariable Regions of Immunoglobulins. *J. Mol. Biol.,* 1987, vol. 196, 901-917